(19) Europäisches Patentamt
European Patent Office
Office européen des brevets

(11) **EP 3 344 309 B1**

(12) **EUROPÄISCHE PATENTSCHRIFT**

(45) Veröffentlichungstag und Bekanntmachung des Hinweises auf die Patenterteilung:
**10.07.2019   Patentblatt 2019/28**

(21) Anmeldenummer: **16767138.7**

(22) Anmeldetag: **31.08.2016**

(51) Int Cl.:
*A61M 1/36* (2006.01)          *A61M 1/34* (2006.01)
*A61M 1/16* (2006.01)          *A61M 5/168* (2006.01)
*G01F 25/00* (2006.01)

(86) Internationale Anmeldenummer:
**PCT/EP2016/001471**

(87) Internationale Veröffentlichungsnummer:
**WO 2017/036598 (09.03.2017 Gazette 2017/10)**

(54) **VERFAHREN ZUR KALIBRIERUNG UND / ODER ÜBERWACHUNG EINES FLUSSSENSORS**

METHOD FOR CALIBRATING AND/OR MONITORING A FLOW SENSOR

PROCÉDÉ D'ÉTALONNAGE ET/OU DE SURVEILLANCE D'UN CAPTEUR DE DÉBIT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.09.2015   DE 102015011424**

(43) Veröffentlichungstag der Anmeldung:
**11.07.2018   Patentblatt 2018/28**

(73) Patentinhaber: **Fresenius Medical Care Deutschland GmbH**
**61352 Bad Homburg (DE)**

(72) Erfinder:
• **NIKOLIC, Dejan**
  **65812 Bad Soden (DE)**
• **PETERS, Arne**
  **61352 Bad Homburg (DE)**
• **WIKTOR, Christoph**
  **63571 Gelnhausen (DE)**

(74) Vertreter: **Behr, Wolfgang**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
**EP-A2- 2 468 390          WO-A1-2015/007596**
**WO-A1-2015/073604**

**Beschreibung**

Die vorliegende Erfindung betrifft ein Verfahren zur Kalibrierung und/oder Überwachung eines Fluss-Sensors eines medizinischen Systems, welcher mit einem Ausgleichsvolumen fluidisch verbunden und/oder verbindbar ist. Weiterhin betrifft die vorliegende Erfindung ein entsprechendes medizinisches System.

Der Einsatz von Fluss-Sensoren hat in der Medizintechnik eine ganze Reihe von Vorteilen. Insbesondere erlauben Fluss-Sensoren eine direkte Messung der Zielgröße, und weisen eine hohe Messgeschwindigkeit auf. Sie werden daher oftmals zur Bilanzierung und/oder Dosierung von Flüssigkeitsmengen eingesetzt. Fluss-Sensoren haben bisher jedoch einen großen Nachteil: Die Überwachung der Messgenauigkeit und die absolute Kalibrierung der Fluss-Sensoren konnte bisher noch nicht zufriedenstellend gelöst werden.

Um den hohen Genauigkeitsansprüchen für Dosieraufgaben in der Dialyse oder der Infusionstechnik gerecht zu werden, wurden gemäß dem Stand der Technik daher statt einfacher Fluss-Sensoren häufig komplexe Systeme eingesetzt. Bekannte Maßnahmen umfassen dabei den redundanten Aufbau der Fluss-Sensoren, d. h. es werden zwei Fluss-Sensoren im selben Strom eingesetzt und deren Messwerte verglichen. Weiterhin mussten schon in der Produktion sehr umfangreiche Kontrollen eingeführt werden. Zudem werden üblicherweise robuste und damit größere und schwerere Fluss-Sensoren eingesetzt.

Aus der WO2015/073604 A1 ist es bekannt, parallel zu einer Druchflussmessung durch einen Flusssensor den Druck in einer Pumpkammer, welche zum Pumpen der medizinischen Flüssigkeit eingesetzt wird, und der Druck in einer Referenzkammer zu messen, um das Gasvolumen in der Pumpkammer zu bestimmen, um hieraus den Durchfluss durch die Pumpkammer zu bestimmen.

Aus der EP 2 468 390 A2 ist es bekannt, einen Flusssensor durch Messen des Füllstandes in einer Messkammer zu kalibrieren.

Aus der WO 2015/007596 A1 ist ein Verfahren zum relativen Kalibrieren zweier Dialysatpumpen bekannt.

Aufgabe der vorliegenden Erfindung ist es, ein Verfahren und ein medizinisches System zur Verfügung zu stellen, welches eine einfache Kalibrierung und/oder Überwachung eines Fluss-Sensors in einem medizinischen System erlaubt.

Diese Aufgabe wird erfindungsgemäß durch ein Verfahren gemäß Anspruch 1 sowie ein System gemäß Anspruch 10 gelöst. Vorteilhafte Ausgestaltungen der vorliegenden Erfindung sind Gegenstand der Unteransprüche.

Die vorliegende Erfindung zeigt dabei in einem ersten Aspekt ein Verfahren zur Kalibrierung und/oder Überwachung eines Fluss-Sensors eines medizinischen Systems, wobei der Fluss-Sensor mit einem Ausgleichsvolumen fluidisch verbunden und/oder verbindbar ist. Das erfindungsgemäße Verfahren weist dabei folgende Schritte auf:

- Einstellen oder Messen eines ersten Druckniveaus,
- Öffnung einer Fluidverbindung, so dass Flüssigkeit durch den Fluss-Sensor aus dem oder in das Ausgleichsvolumen fließt,
- Messen einer durch den Flüssigkeitssensor fließenden ersten Flüssigkeitsmenge V3 mittels des Fluss-Sensors,
- Einstellen oder Messen eines zweiten Druckniveaus,
- Bestimmen der ersten Flüssigkeitsmenge V3 anhand des ersten und des zweiten Druckniveaus und
- Kalibrierung und/oder Überwachung des Fluss-Sensors anhand der mittels des Fluss-Sensors gemessenen ersten Flüssigkeitsmenge und der anhand des ersten und des zweiten Druckniveaus bestimmten ersten Flüssigkeitsmenge.

Die vorliegende Erfindung erlaubt durch die Bestimmung der ersten Flüssigkeitsmenge anhand der beiden Druckniveaus eine ebenso einfache wie zuverlässige Absolutkalibrierung und/oder Überwachung des Fluss-Sensors. Hierdurch können einfache Fluss-Sensoren eingesetzt werden, und damit Kosten, Gewicht und Bauraum eingespart werden. Dennoch wird die für den Einsatz in der Medizintechnik notwendige Genauigkeit erreicht. Insbesondere kann der erfindungsgemäße Fluss-Sensor dabei in einem Dialyse- und/oder Infusionssystem eingesetzt werden, insbesondere für Dosier- und/oder Bilanzieraufgaben.

In einer möglichen Ausführungsform kann die vorliegende Erfindung dabei zur Kalibrierung und/oder Überwachung eines einkanaligen Fluss-Sensors eingesetzt werden, welcher bevorzugt zur Dosierung medizinscher Flüssigkeiten verwendet wird. In einer zweiten Ausführungsform kann die vorliegende Erfindung eingesetzt werden, um zumindest einen Kanal eines zweikanaligen Bilanzier-Fluss-Sensors absolut zu kalibrieren und/oder zu überwachen. Der zweite Kanal eines solchen zweikanaligen Bilanzier-Fluss-Sensors kann dann entweder ebenfalls durch das erfindungsgemäße Verfahren kalibriert und/oder überwacht werden, und/oder der erste erfindungsgemäß kalibrierte und/oder überwachte Kanal des Bilanzier-Fluss-Sensors kann als Bezugsgröße für die Kalibrierung und/oder Überwachung des zweiten Kanals eingesetzt werden.

In einer bevorzugten Ausführungsform der vorliegenden Erfindung wird dabei ein mit dem Ausgleichsvolumen gekoppeltes starres Referenzvolumen eingesetzt, wobei die Bestimmung der Flüssigkeitsmenge anhand des bekannten Volumeninhalts des Referenzvolumens und mindestens einer Druckmessung im Referenzvolumen erfolgt. Der Einsatz eines Referenzvolumens erlaubt dabei eine hochgenaue Bestimmung der Flüssigkeitsmenge und damit eine hochgenaue Kalibrierung und Überwachung des Fluss-Sensors.

Bevorzugt ist das Referenzvolumen dabei mit Gas gefüllt. Weiter bevorzugt steht das Referenzvolumen dabei über eine verschließbare Druckverbindung mit dem Ausgleichsvolumen in Verbindung.

Das Ausgleichsvolumen ist bevorzugt teilweise mit Flüssigkeit und teilweise mit Gas gefüllt. Änderungen der Flüssigkeitsmenge im Ausgleichsvolumen haben daher eine Druckänderung im Ausgleichsvolumen zur Folge. Wird oder ist dabei die Druckverbindung zum Referenzvolumen geöffnet, hat die Änderungen der Flüssigkeitsmenge im Ausgleichsvolumen eine Veränderung des Druckes im Referenzvolumen zur Folge. Aufgrund des bekannten Volumeninhalts des Referenzvolumens und einer Druckmessung lassen sich so anhand des Gasgesetzes Änderungen der Flüssigkeitsmenge im Ausgleichsvolumen berechnen. Bevorzugt ist die Druckverbindung und/oder das medizinische System dabei so ausgestaltet, dass ein Flüssigkeitsübertritt zwischen dem Ausgleichsvolumen und dem Referenzvolumen vermieden wird.

Besteht das medizinische System dabei aus einem Disposable und einer Behandlungsmaschine, so ist das starre Referenzvolumen bevorzugt maschinenseitig vorgesehen, das Ausgleichsvolumen dagegen bevorzugt zumindest teilweise disposableseitig. In einer alternativen Ausführungsform kann jedoch auch das Referenzvolumen disposableseitig angeordnet sein.

Gemäß dem erfindungsgemäßen Verfahren wird bevorzugt eine Druckverbindung zwischen dem Ausgleichsvolumen und dem Referenzvolumen mindestens einmal geschlossen und/oder geöffnet. Durch das Öffnen der Druckverbindung kann ein Druckausgleich zwischen dem Ausgleichsvolumen und dem Referenzvolumen erfolgen. Durch das Schließen der Druckverbindung können dagegen im Ausgleichsvolumen und im Referenzvolumen unterschiedliche Drücke hergestellt werden.

Bevorzugt ist oder wird die Druckverbindung zwischen dem Ausgleichsvolumen und dem Referenzvolumen dabei geöffnet, während oder nachdem Flüssigkeit durch den Fluss-Sensor aus dem oder in das Ausgleichsvolumen fließt. Insbesondere führt dabei die Änderungen der Flüssigkeitsmenge im Ausgleichsvolumen durch das Ab- oder Zufließen der Flüssigkeit über die geöffnete Druckverbindung zu einer Druckänderung im Referenzvolumen.

Weiterhin kann der Druck im Ausgleichsvolumen und/oder im Referenzvolumen vor und nach dem Öffnen und/oder Schließen der Druckverbindung bestimmt werden.

In einer weiteren bevorzugten Ausführungsform wird der Druck im Ausgleichsvolumen und/oder im Referenzvolumen vor und nach dem Abfließen der ersten Flüssigkeitsmenge in das oder aus dem Ausgleichsvolumen bestimmt. Bevorzugt wird dabei anhand der beiden Druckwerte vor und nach dem Abfließen eine Druckänderung bestimmt.

Alternativ oder zusätzlich kann die durch den Fluss-Sensor fließende erste Flüssigkeitsmenge anhand der Druckänderung im Ausgleichsvolumen und/oder im Referenzvolumen bestimmt werden. Insbesondere kann dabei eine Druckänderung im Referenzvolumen eingesetzt werden, um die durch den Flüssigkeitssensor fließende erste Flüssigkeitsmenge zu bestimmen.

Weiterhin alternativ oder zusätzlich kann die durch den Flüssigkeitssensor fließende erste Flüssigkeitsmenge anhand einer Druckänderung und mindestens einem absoluten Druckwert bestimmt werden. Allein die Druckänderung reicht dabei üblicherweise nicht aus, um die durch den Flüssigkeitssensor abgeflossene erste Flüssigkeitsmenge zu bestimmen. Daher kann zur Bestimmung der ersten Flüssigkeitsmenge weiterhin mindestens ein absoluter Druckwert eingesetzt werden. In einer möglichen Ausführungsform können auch mehrere, während unterschiedlicher Phasen und/oder in unterschiedlichen Bereichen bestimmte absolute Druckwerte in die Berechnung der ersten Flüssigkeitsmenge eingehen.

Gemäß der vorliegenden Erfindung erfolgt eine Beaufschlagung des Ausgleichsvolumens mit Druck mittels einer Pumpe des medizinischen Systems. Dabei handelt es sich erfindungsgemäß um eine Pumpe, welche ohnehin vorhanden ist, um eine medizinische Flüssigkeit innerhalb des medizinischen Systems zu bewegen.

Bei der Pumpe kann es sich dabei insbesondere um
jene Pumpe handeln, welche die Flüssigkeit im Normalbetrieb des Systems durch den Flusssensor transportiert, sodass der Flusssensor zum Messen der durch die Pumpe gepumpten Flüssigkeit eingesetzt werden kann. Bevorzugt wird dabei zur Beaufschlagung des Ausgleichsvolumens mit Druck die Fluidverbindung geschlossen, sodass die von der Pumpe in das Ausgleichsvolumen gepumpte Flüssigkeit nicht wie üblich abfließen kann und daher den Druck im Ausgleichsvolumen erhöht.

Die Pumpe kann dabei über eine Fluidverbindung mit dem Ausgleichsvolumen verbunden und / oder verbindbar sein. Insbesondere kann die Pumpe dabei ein zum Ausgleichsvolumen separates Bauelement bilden, welches mit diesem über eine Fluidverbindung in Verbindung steht. Beispielsweise kann es sich bei der erfindungsgemäßen Pumpe um eine okkludierende Pumpe, insbesondere um eine Rollenpumpe handeln. Dabei kann ein Pumpsegment vorgesehen sein, welches in die Rollenpumpe einlegbar ist und mit dem Ausgleichsvolumen in Fluidverbindung steht.

Die vorliegende Erfindung kann dabei sowohl dann eingesetzt werden, wenn die flüssigkeitsführenden Bereiche und insbesondere das Ausgleichsvolumen flexibel ausgestaltet sind, und daher auf Druckänderungen mit einer Änderung ihres Volumeninhalts antworten, als auch dann, wenn die flüssigkeitsführenden Bereiche starr ausgebildet sind. Bevorzugt werden für diese beiden Anwendungsfälle jedoch unterschiedliche Verfahrensabläufe gewählt.

In einer ersten Ausführungsform, welche insbesondere dann zum Einsatz kommt, wenn es sich bei den flüssigkeitsführenden Bereichen um ein starres System handelt, können dabei folgende Schritte vorgesehen sein:
In einem ersten Schritt kann ein Druck p1 in dem Ausgleichsvolumen erzeugt werden, während die Druckverbindung zum Referenzvolumen getrennt ist und dort ein Druck p0 gemessen wird. In einem zweiten Schritt kann die Druckverbindung zwischen dem Ausgleichsvolumen und dem Referenzvolumen geöffnet werden, sodass sich in dem Ausgleichsvolumen und dem Referenzvolumen ein gemeinsamer Druck p2 einstellt. In einem dritten Schritt kann dann die Fluidverbindung geöffnet werden, sodass Flüssigkeit durch den Flusssensor aus dem oder in das Ausgleichsvolumen fließt, wobei die durch den Flusssensor fließende erste Flüssigkeitsmenge V3 mittels des Flusssensors gemessen wird und der sich durch das Zu- oder Abfließen der ersten Flüssigkeitsmenge V3 in dem Ausgleichsvolumen und dem Referenzvolumen einstellende Druck p3 gemessen wird.

Bevorzugt wird dabei die erste Flüssigkeitsmenge V3 aus den Drücken p0, p1, p2 und p3 sowie dem Volumeninhalt Vref des Referenzvolumens bestimmt. Bevorzugt gilt dabei:

$$V3 = Vref * (p2 - p3) / p3 * (1 + (p0 - p2) / (p2 - p1))$$

Ein solches Verfahren kann insbesondere dann eingesetzt werden, wenn die Druckänderung von p1 auf p2 und / oder auf p3 keinen Einfluss auf den Volumeninhalt des Ausgleichsvolumens hat.

Gemäß einer zweiten Verfahrensvariante kann die Fluidverbindung auch solange geöffnet werden, bis sich in dem Ausgleichsvolumen durch Zu- oder Abfließen einer Flüssigkeitsmenge ein bestimmter Druck eingestellt hat, wobei es sich bei der Flüssigkeitsmenge, welche zu- oder abgeflossen ist, bis sich ein bestimmter Druck eingestellt hat, bevorzugt um die erste Flüssigkeitsmenge handelt. Hierdurch kann der Druck, bei welchem die Messung durchgeführt wird, vorgegeben werden, um eventuelle Einflüsse der Flexibilität des Ausgleichsvolumens auf das Messergebnis zu verhindern.

Bei dem bestimmten Druck kann es sich dabei entweder um einen vorgegebenen, in einer Maschinensteuerung fest abgelegten Druck handeln, oder einen Druck, bei welchem zuvor ein Messschritt durchgeführt wurde.

Bevorzugt kann dabei in einem ersten Schritt ein Druck p1 in dem Ausgleichsvolumen erzeugt werden, während die Druckverbindung zum Referenzvolumen geöffnet ist. Weiterhin kann in einem zweiten Schritt die Druckverbindung zwischen dem Ausgleichsvolumen und dem Referenzvolumen geschlossen und die Fluidverbindung geöffnet werden, sodass eine zweite Flüssigkeitsmenge V2 durch den Flusssensor aus dem oder in das Ausgleichsvolumen fließt, wobei der sich durch das Zu- oder Abfließen der zweiten Flüssigkeitsmenge V2 in dem Ausgleichsvolumen einstellende Druck p2 gemessen wird. Weiterhin kann in einem dritten Schritt die Druckverbindung zwischen dem Ausgleichsvolumen und dem Referenzvolumen geöffnet werden und die Fluidverbindung solange geöffnet werden, bis sich in dem Ausgleichsvolumen und dem Referenzvolumen durch Abfließen der ersten Flüssigkeitsmenge V3 wieder der Druck p2 eingestellt hat.

Dabei handelt es sich bei der ersten Flüssigkeitsmenge V3 um jene Menge, welche über den Flusssensor gemessen sowie anhand der Druckniveaus bestimmt wird, um den Flusssensor zu kalibrieren und / oder zu überwachen.

Insbesondere kann dabei die erste Flüssigkeitsmenge V3 aus den Drücken p1 und p2 und dem Volumeninhalt Vref des Referenzvolumens bestimmt werden. Bevorzugt gilt dabei folgende Formel:

$$V3 = Vref * (p1 - p2) / p2$$

Die zweite Verfahrensvariante kann dabei auch dann eingesetzt werden, wenn das Ausgleichsvolumen flexibel ausgestaltet ist, sodass Druckänderungen einen Einfluss auf den Volumeninhalt des Ausgleichsvolumens haben. Denn durch den gemäß der zweiten Variante vorgesehenen Verfahrensablauf erfolgen die beiden Messungen vor und nach dem Abfließen der ersten Flüssigkeitsmenge V3 jeweils beim gleichen Druck p2, sodass eine eventuelle Veränderung des Ausgleichsvolumens aufgrund des herrschenden Druckes sich zu den beiden Messzeitpunkten in gleicher Weise auswirkt und daher auf das Ergebnis keinen Einfluss hat.

Die vorliegende Erfindung umfasst in einem zweiten, von dem erfindungsgemäßen Verfahren unabhängigen Aspekt ein medizinisches System mit einer Behandlungsmaschine und einem Disposable mit flüssigkeitsführenden Bereichen, welches an die Behandlungsmaschine ankoppelbar ist. Das Disposable weist dabei einen Flusssensor-Abschnitt auf. Erfindungsgemäß ist dabei vorgesehen, dass das Disposable weiterhin ein Ausgleichsvolumen aufweist, welches mit dem Flusssensor-Abschnitt fluidisch verbunden und / oder verbindbar ist. In einer ersten Ausführungsvariante ist das Ausgleichsvolumen an ein Referenzvolumen der Behandlungsmaschine ankoppelbar. In einer zweiten Ausführungsvariante weist das Disposable ein Referenz-Volumen auf, welches mit dem Ausgleichsvolumen über eine Druckverbindung verbunden oder verbindbar ist. Durch das erfindungsgemäße Ausgleichsvolumen sowie das Referenzvolumen kann der Flusssensor des medizinischen Systems einfach und dennoch sehr genau kalibriert und / oder überwacht werden.

Das Disposable kann dabei beispielsweise eine Kassette mit einem Hartteil, in welchem die flüssigkeitsführenden Bereiche eingelassen sind, umfassen. Bevorzugt wird das Hartteil dabei von einer flexiblen Folie abgedeckt, wobei das Disposable über die flexible Folie mit einer Ankoppelfläche der Behandlungsmaschine koppelbar ist. Alternativ oder zusätzlich kann das Disposable ein Schlauchset umfassen oder aus einem solchen bestehen. Weiterhin kann es sich bei dem Disposable um eine Anordnung aus einem oder mehreren Schlauch- und / oder Kassettenelementen handeln.

Das Disposable und/oder die das Ausgleichsvolumen bildenden Bestandteile des Disposables können dabei in einer ersten Ausführungsform starr ausgebildet sein, und in einer zweiten Ausführungsform zumindest teilweise flexibel ausgestaltet sein. Je nach Ausführungsform wird dabei bevorzugt eine der oben beschriebenen Verfahrensvarianten zur Kalibrierung und Überwachung eingesetzt.

Bevorzugt weist das Disposable weiterhin einen Pumpabschnitt auf, welcher mit dem Ausgleichsvolumen fluidisch verbunden oder verbindbar ist. Bevorzugt handelt es sich bei dem Pumpabschnitt dabei um einen in eine okkludierende Pumpe der Behandlungsmaschine einlegbaren Schlauchabschnitt. Der Pumpabschnitt kann dabei beispielsweise mit einer Kassette, in welcher das Ausgleichsvolumen und / oder der Flusssensorabschnitt angeordnet sind, verbunden sein.

Bevorzugt weist die Behandlungsmaschine mindestens einen und bevorzugt mindestens zwei Drucksensoren auf, bei denen bzw. über die der Druck im Ausgleichsvolumen und / oder im Referenzvolumen messbar ist. Insbesondere kann das Ausgleichsvolumen dabei an eine Ankoppelfläche der Behandlungsmaschine ankoppelbar sein, in welcher der Drucksensor angeordnet ist. Alternativ kann der Druck im Ausgleichsvolumen des Disposables auch in einem an das Ausgleichsvolumen gekoppelten Ausgleichsvolumen der Behandlungsmaschine gemessen werden. Das Referenzvolumen ist in einer ersten Variante bevorzugt im Bereich der Behandlungsmaschine angeordnet und weist einen dem Referenzvolumen zugeordneten Drucksensor auf. Das Referenzvolumen ist in einer zweiten Variante bevorzugt im Bereich des Disposables angeordnet und kann mit einem im Bereich der Behandlungsmaschine angeordneten Drucksensor gekoppelt werden.

Weiterhin kann die Behandlungsmaschine mindestens einen Ventil- und / oder Klemmenaktor aufweisen, über welchen eine Fluidverbindung im Disposable, über welche Flüssigkeit durch den Flusssensor aus dem oder in das Ausgleichsvolumen fließen kann, geschlossen und geöffnet werden kann. Hierdurch kann die Steuerung der Behandlungsmaschine durch Ansteuerung des Ventil- und / oder Klemmenaktors die Fluidverbindung im Disposable öffnen oder schließen.

Weiterhin kann die Behandlungsmaschine mindestens einen Ventil- und / oder Klemmenaktor aufweisen, über welchen eine Druckverbindung zwischen dem Ausgleichsvolumen und dem Referenzvolumen geöffnet und geschlossen werden kann. Hierdurch kann die Steuerung durch Ansteuerung des entsprechenden Ventil- und / oder Klemmenaktors das erfindungsgemäße Verfahren, bei welchem die Druckverbindung zwischen dem Ausgleichsvolumen und dem Referenzvolumen geöffnet bzw. geschlossen werden muss, durchführen.

Weiterhin kann die Behandlungsmaschine mindestens einen Ventil- und / oder Klemmenaktor aufweisen, über welchen eine Druckverbindung zwischen dem Referenzvolumen und einem Druckbereich, insbesondere der Atmosphäre, geöffnet und geschlossen werden kann. Hierdurch kann das Referenzvolumen entlüftet werden.

Weiterhin bevorzugt weist die Behandlungsmaschine mindestens einen Flusssensor auf, welcher mit dem Flusssensor-Abschnitt des Disposables koppelbar ist.

Bei dem Flusssensor kann es sich um einen Ultraschallflusssensor handeln, welcher an einen den Flusssensor-Abschnitt des Disposables bildenden Strömungskanal und / oder Schlauchabschnitt des Disposables ankoppelbar ist. Alternativ kann es sich bei dem Flusssensor um einen Druckdifferenzsensor handeln, dessen Sensoren jeweils an eine Messstelle vor und nach einer Messstrecke und insbesondere einer Messblende des Disposables koppelbar sind. Weiterhin kann es sich bei dem Flusssensor um einen magnetisch-induktiven Sensor handeln, wobei bevorzugt Spulen und/oder Messelektroden des Behandlungsgerätes an ein Messrohr des Disposables ankoppelbar sind.

In einer ersten Ausführungsform kann das Ausgleichsvolumen des Disposables über eine Fluidverbindung mit dem Referenzvolumen verbindbar sein, wobei die Verbindung bevorzugt durch das Ankoppeln des Disposables an die Behandlungsmaschine erfolgt. Bevorzugt weist das Disposable dabei einen mit dem Ausgleichsvolumen in Verbindung stehenden Fluidkanal auf, welcher mit einem Fluidkanal der Behandlungsmaschine koppelbar ist, der mit dem Referenzvolumen in Verbindung steht.

Bevorzugt ist dabei in dem Fluidkanal des Disposables ein Sterilfilter vorgesehen, welcher einen Flüssigkeitsübertritt in die Behandlungsmaschine verhindert, aber gasdurchlässig ist. Insbesondere kann es sich um einen hydrophoben Sterilfilter handeln. Alternativ oder zusätzlich kann die Steuerung der Behandlungsmaschine durch eine entsprechende Druck- und/oder Füllniveausteuerung einen Flüssigkeitsübertritt von dem Disposable in die Behandlungsmaschine verhindern. Bevorzugt ist in einem solchen Ausführungsfall das Ausgleichsvolumen des Disposables teilweise mit Flüssigkeit und teilweise mit Gas gefüllt. Eine Volumenänderung der Flüssigkeit im Ausgleichsvolumen führt so zu einer Volumenänderung des Gases im Ausgleichsvolumen und damit einer entsprechenden Druckänderung.

In einem zweiten Ausführungsbeispiel kann das Ausgleichsvolumen des Disposables eine Membran aufweisen, über welche es mit einem Ausgleichsvolumen der Behandlungsmaschine koppelbar ist, das wiederum mit dem Referenzvolumen fluidisch in Verbindung steht. Bevorzugt ist in einem solchen Ausführungsfall das Ausgleichsvolumen des Disposables teilweise oder komplett mit Flüssigkeit gefüllt, das Ausgleichsvolumen der Behandlungsmaschine dagegen mit Gas. Bevorzugt sind beide Ausgleichsvolumina jeweils über Membranen verschlossen, wobei die Membranen aneinander gekoppelt sind. Eine Volumenänderung der Flüssigkeit im Ausgleichsvolumen des Disposables führt so zu einer Volumenänderung des Gases im Ausgleichsvolumen der Behandlungsmaschine, und damit einer entsprechenden Druckänderung.

In einem dritten Ausführungsbeispiel kann das Referenzvolumen im Disposable angeordnet sein und insbesondere durch eine Kammer im Hartteil des Disposables gebildet werden. Die Kammer kann dabei zur Behandlungsmaschine hin durch die flexible Folie abgeschlossen sein. Da die Folie im Betrieb an der Ankoppelfläche der Behandlungsmaschine anliegt, bildet die Folie dennoch eine starre Wand des Referenzvolumens. Alternativ kann die Kammer auf allen Seiten durch das Hartteil begrenzt sein. Die Druckverbindung zu dem Ausgleichsvolumen kann bevorzugt wie beim ersten bzw. zweiten Ausführungsbeispiel durch eine Fluidverbindung oder über eine Membran erfolgen.

Ist die Druckverbindung zwischen dem Ausgleichsvolumen und dem Referenzvolumen dabei geöffnet, sind von der Druckänderung sowohl das Ausgleichsvolumen, als auch das Referenzvolumen betroffen.

Bei dem medizinischen System handelt es sich dabei bevorzugt um ein Dialysesystem und / oder um ein Infusionssystem. Bevorzugt wird der Pumpabschnitt dabei dazu eingesetzt, um Dialysat und / oder Blut zu pumpen, und / oder um eine Infusionslösung zu pumpen. Insbesondere kann es sich dabei um ein Hämodialysesystem oder ein Peritonealdialysesystem handeln.

Der Flusssensor wird dabei bevorzugt zur Dosierung und / oder Bilanzierung der gepumpten medizinischen Flüssigkeit, insbesondere des Blutes, des Dialysats und / oder der Infusionslösung eingesetzt. Bevorzugt steht der Flusssensor dabei mit einer Steuerung der Behandlungsmaschine in Verbindung, wobei die Steuerung die Signale des Flusssensors auswertet, um die gepumpte Flüssigkeitsmenge zu bestimmen.

Bei dem Flusssensor-Abschnitt und dem Flusssensor kann es sich dabei um einen Kanal eines einkanaligen Flusssensors, zum Beispiel eines MID-Sensors handeln, welcher bevorzugt zur Dosierung medizinischer Flüssigkeiten eingesetzt wird. Alternativ kann es sich bei dem Flusssensor-Abschnitt und dem Flusssensor um einen Kanal eines zweikanaligen Bilanzierflusssensors handeln.

Unabhängig von der konkreten Ausgestaltung des medizinischen Systems wird bevorzugt das erfindungsgemäße Verfahren dazu eingesetzt, um den Flusssensor zu kalibrieren und / oder zu überwachen.

Die vorliegende Erfindung umfasst dabei in einem dritten Aspekt, welcher unabhängig von dem zweiten Aspekt ist, ein medizinisches System mit einer Steuerung zur Auswertung der Signale eines Flusssensors, wobei die Steuerung eine Kalibrierungs- und/oder Überwachungsfunktion zur Kalibrierung und/oder Überwachung des Flusssensors gemäß dem oben beschriebenen erfindungsgemäßen Verfahren aufweist.

Bevorzugt wird die Kalibrierung und/oder Überwachung dabei automatisch von der Steuerung vorgenommen. Alternativ oder zusätzlich kann das erfindungsgemäße Verfahren zur Kalibrierung und / oder Überwachung automatisch von der Steuerung durchgeführt werden.

Insbesondere kann die Steuerung hierfür eine Pumpe und / oder Ventil und / oder Klemmenaktoren ansteuern und Messwerte von Druckmesssensoren auswerten, um ein erfindungsgemäßes Verfahren durchzuführen.

Bei dem erfindungsgemäßen System gemäß dem dritten Aspekt mit der erfindungsgemäßen Steuerung zur Durchführung des Verfahrens handelt es sich dabei bevorzugt um ein medizinisches System, wie es oben gemäß dem zweiten Aspekt beschrieben wurde.

Die vorliegende Erfindung umfasst weiterhin ein Disposable für ein medizinisches System gemäß dem zweiten Aspekt, wie es oben beschrieben wurde. Insbesondere weist das Disposable dabei einen Flusssensor-Abschnitt sowie ein Ausgleichsvolumen auf, welches mit dem Flusssensor-Abschnitt fluidisch verbunden und / oder verbindbar ist, wobei das Ausgleichsvolumen an ein Referenzvolumen der Behandlungsmaschine ankoppelbar ist oder wobei das Disposable ein Referenz-Volumen aufweist, welches mit dem Ausgleichsvolumen über eine Druckverbindung verbunden oder verbindbar ist. Bevorzugt ist das Disposable dabei so aufgebaut, wie dies oben im Hinblick auf das erfindungsgemäße System näher beschrieben wurde.

Weiterhin umfasst die vorliegende Erfindung eine Behandlungsmaschine für ein medizinisches System gemäß dem zweiten oder dem dritten Aspekt, wie sie oben dargestellt wurden. Insbesondere weist die Behandlungsmaschine dabei gemäß dem zweiten Aspekt einen Flusssensor auf, welcher an den Flusssensor-Abschnitt eines Disposables ankoppelbar ist. Die Behandlungsmaschine kann weiterhin ein Referenzvolumen aufweisen, welches an ein Ausgleichsvolumen des Disposables ankoppelbar ist, oder einen Drucksensor, welcher an ein Referenzvolumen des Disposables ankoppelbar ist. Gemäß dem dritten Aspekt weist die Behandlungsmaschine eine Steuerung zur Auswertung der Signale eines Flusssensors auf, wobei die Steuerung eine Kalibrierungs- und/oder Überwachungsfunktion zur Kalibrierung und/oder Überwachung des Flusssensors gemäß einem erfindungsgemäßen Verfahren aufweist.

Bevorzugt ist die Behandlungsmaschine dabei so ausgestaltet, wie dies bereits oben näher dargestellt wurde.

Die vorliegende Erfindung wird nun anhand von Ausführungsbeispielen sowie Zeichnungen näher dargestellt.

Dabei zeigen:

Fig. 1: ein erstes Ausführungsbeispiel eines erfindungsgemäßen medizinischen Systems, mit welchem ein erfindungsgemäßes Verfahren durchgeführt werden kann,

Fig. 2a bis 2d: mehrere Schritte eines ersten Ausführungsbeispiels eines erfindungsgemäßen Verfahrens,

Fig. 3a bis 3e: mehrere Schritte eines zweiten Ausführungsbeispiels eines erfindungsgemäßen Verfahrens,

Fig. 4: den Druckverlauf im Ausgleichsvolumen bei dem in Fig. 3a bis 3e dargestellten zweiten Ausführungsbeispiel eines erfindungsgemäßen Verfahrens, und

Fig. 5: ein zweites Ausführungsbeispiel eines erfindungsgemäßen Systems, bei welchem ein erfindungsgemäßes Verfahren zum Einsatz kommen kann.

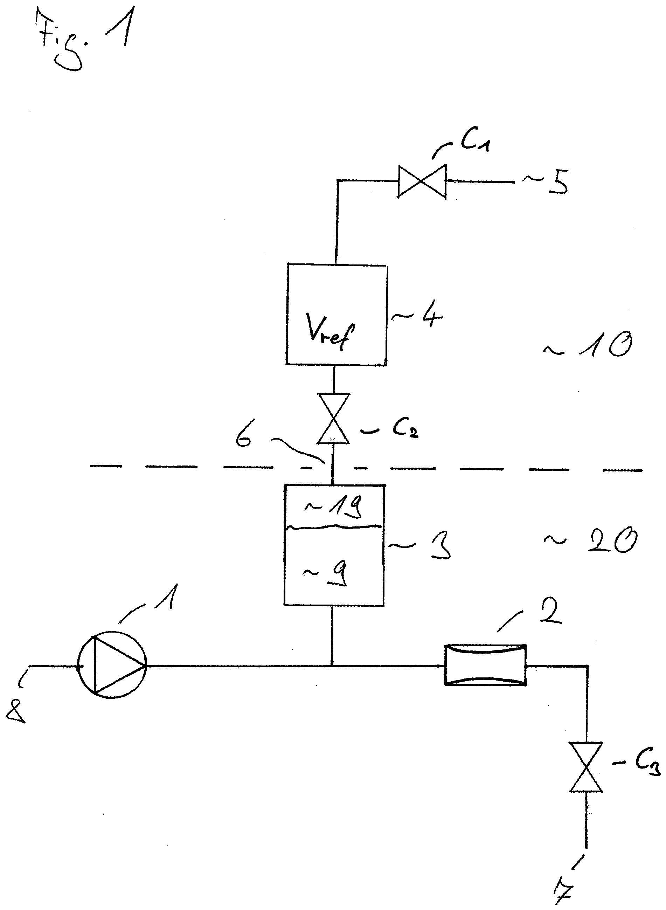

In Fig. 1 ist ein erstes Ausführungsbeispiel eines erfindungsgemäßen medizinischen Systems gezeigt. Das medizinische System weist einen flüssigkeitsführenden Bereich 20 auf, welcher dem Transport einer medizinischen Flüssigkeit dient und beispielsweise als Disposable ausgeführt sein kann.

Der flüssigkeitsführende Bereich 20 weist dabei einen Pumpabschnitt 1 und einen Flusssensor-Abschnitt 2 auf, welche fluidisch miteinander verbunden sind. Der Pumpabschnitt 1 wird dabei im Normalbetrieb des medizinischen Systems dazu eingesetzt, um Flüssigkeit von einer Flüssigkeitsquelle 8 durch den Flusssensor 2 zu einem Flüssigkeitsziel 7 zu pumpen. Dabei wird der Flusssensor-Abschnitt 2 dazu eingesetzt, die durch den Pumpabschnitt 1 gepumpte Flüssigkeitsmenge zu bestimmen.

Bei dem erfindungsgemäßen medizinischen System kann es sich insbesondere um ein Dialysesystem und / oder ein Infusionssystem handeln. Insbesondere kann der fluidführende Bereich 20 und damit der Pumpabschnitt 1 und der Flusssensor-Abschnitt 2 dazu eingesetzt werden, um eine medizinische Flüssigkeit wie Blut, Dialysat und / oder eine Infusionslösung zu pumpen. Insbesondere kann es sich bei dem medizinischen System dabei um ein Hämodialysesystem oder ein Peritonealdialysesystem handeln.

Bei dem Flusssensor-Abschnitt 2 kann es sich in einer ersten möglichen Ausfürhungsform um einen Kanal eines einkanaligen Flusssensors handeln. Der Flusssensor wird in diesem Fall dazu eingesetzt, um die durch ihn hindurch fließende medizinische Flüssigkeit zu dosieren. Alternativ kann es sich bei dem in Fig. 1 gezeigten Flusssensor-Abschnitt 2 auch um einen Kanal eines zweikanaligen Bilanzier-Flusssensors handeln, wobei der zweite Kanal in Fig. 1 nicht näher dargestellt ist. Der Bilanzier-Flusssensor wird in diesem Fall dazu eingesetzt, um die Abgabe und Entnahme einer medizinischen Flüssigkeit zu bilanzieren.

Der flüssigkeitsführende Bereich 20 ist wie in Fig. 1 durch die gestrichelte Linie dargestellt an eine Behandlungsmaschine 10 ankoppelbar. Die Behandlungsmaschine umfasst dabei bevorzugt alle Aktoren zur Steuerung der Fluidströme und alle elektronischen Komponenten der Sensoren. Bevorzugt ist dabei der Pumpabschnitt 1 des flüssigkeitsführenden Bereiches an einen nicht näher dargestellten Pumpaktor der Behandlungsmaschine ankoppelbar. Insbesondere kann es sich bei dem Pumpabschnitt 1 dabei um ein Pumpschlauchsegment handeln, welches in eine Rollenpumpe der Behandlungsmaschine einlegbar ist. Weiterhin kann der Flusssensor-Abschnitt 2 des flüssigkeitsführenden Bereiches an einen Flusssensor der Behandlungsmaschine angekoppelt werden. Bei dem Flusssensor kann es sich dabei um einen Ultraschall-Flusssensor handeln, welcher an einen flüssigkeitsführenden Kanal oder Schlauchabschnitt des flüssigkeitsführenden Bereiches ankoppelbar ist. Alternativ kann es sich auch um einen Differenzdruck-Flusssensor handeln, dessen beide Drucksensoren an zwei Druckmessstellen vor und nach einer Messblende des flüssigkeitsführenden Bereiches an den flüssigkeitsführenden Bereich ankoppelbar sind. Bevorzugt handelt es sich bei dem Flusssensor jedoch um einen magnetisch-induktiven Sensor, dessen Spulen und Sensorelektroden an ein Messröhrchen des flüssigkeitsführenden Bereiches ankoppelbar sind.

Bei dem flüssigkeitsführenden Bereich 20 kann es sich wie bereits oben dargestellt bevorzugt um ein Disposable handeln. Beispielsweise kann es sich dabei um eine Kassette handeln, welche an eine Ankoppelfläche der Behandlungsmaschine ankoppelbar ist, um die Aktoren und Sensoren der Behandlungsmaschine mit den entsprechenden Bereichen der Kassette in Verbindung zu bringen. An der Kassette können dabei gegebenenfalls Schlauchabschnitte angeordnet sein, beispielsweise ein Pumpschlauchabschnitt und / oder zu- und abführende Schlauchabschnitte. Alternativ kann es sich bei dem flüssigkeitsführenden Bereich 20 auch um ein Schlauchsystem mit entsprechenden Ankoppelstellen an die Behandlungsmaschine handeln.

Um eine Kalibrierung und Überwachung des Flusssensors zu ermöglichen, weist das flüssigkeitsführende System 20 wie in Fig. 1 dargestellt ein Ausgleichsvolumen 3 auf, welches fluidisch mit dem Pumpabschnitt 1 und dem Flusssensor-Abschnitt 2 in Verbindung steht. Im Ausführungsbeispiel ist das Ausgleichsvolumen 3 dabei zwischen dem Pumpabschnitt 1 und dem Flusssensor-Abschnitt 2 angeordnet. Das Ausgleichsvolumen kann daher mit Druck beaufschlagt werden. Wird dann Flüssigkeit aus dem Ausgleichsvolumen durch den Flusssensor abgelassen, führt dies zu einer Druckänderung im Ausgleichsvolumen, aus welcher die Flüssigkeitsmenge berechnet werden kann, die durch den Flusssensor geflossen ist.

Zur Durchführung der Kalibrierung und Überwachung des Flusssensors ist das Ausgleichsvolumen 3 dabei teilweise mit Flüssigkeit 9, und teilweise mit Gas 19 befüllt. Bei dem Gas handelt es sich dabei insbesondere um Luft.

Das Ausgleichsvolumen 3 ist im Ausführungsbeispiel komplett innerhalb des flüssigkeitsführenden Bereiches 20 angeordnet, und kann über eine lösbare Fluidverbindung 6 mit einem Referenzvolumen 4 der Behandlungsmaschine 10 fluidisch gekoppelt werden. Der gesamte mit dem Ausgleichsvolumen 3 in Verbindung stehende maschinenseitige Bereich ist dabei ebenfalls mit Gas und bevorzugt mit Luft gefüllt. Um einen Flüssigkeitsübertritt von der fluidführenden Seite zur Behandlungsmaschine 10 zu verhindern, kann in der Fluidverbindung 6 bevorzugt im Bereich des fluidführenden Bereiches 20 ein Sterilfilter vorgesehen sein, welcher für Flüssigkeiten undurchlässig ist, jedoch einen Gasübertritt erlaubt, insbesondere ein hydrophober Sterilfilter. In einer alternativen, nicht dargestellten Ausführungsform kann das Referenzvolumen 4 jedoch auch innerhalb des flüssigkeitsführenden Bereiches 20 angeordnet sein und insbesondere einen Teil des Disposables bilden.

Weiterhin sind Ventile oder Klemmen C1 bis C3 vorgesehen, um die einzelnen Bereiche voneinander zu trennen oder miteinander zu verbinden. Das Ventil C1 ist dabei in einer Fluidleitung zwischen dem Referenzvolumen 4 und einem Druckbereich 5, insbesondere der Atmosphäre, angeordnet und erlaubt ein Entlüften des Referenzvolumens 4. Das Ventil C2 ist in der Fluidleitung zwischen dem Referenzvolumen 4 und dem Ausgleichsvolumen 3 angeordnet, wobei das Ventil C2 bevorzugt maschinenseitig vorgesehen ist. Das Ventil oder die Klemme C3 erlaubt es, eine Fluidverbindung innerhalb des flüssigkeitsführenden Bereiches 20 zu öffnen und zu schließen, durch welche der Fluss durch den Flusssensor 2 angesteuert werden kann. Im Ausführungsbeispiel ist das Ventil bzw. die Klemme C3 stromabwärts des Flusssensor-Abschnittes 2 vorgesehen.

Ist die durch das Ventil oder die Klemme C3 gesteuerte Fluidverbindung geschlossen, kann keine Flüssigkeit mehr durch den Flusssensor 2 fließen, sodass die Flüssigkeitsmenge 9 im Ausgleichsvolumen durch Pumpen mit der Pumpe 1 erhöht werden kann. Hierdurch wird der Gasanteil 19 im Ausgleichsvolumen 3 komprimiert und damit der Druck erhöht. Wird umgekehrt die Fluidverbindung über das Ventil oder die Klemme C3 geöffnet, sodass Flüssigkeit über den Flusssensor 2 abfließen kann, verringert sich das Flüssigkeitsvolumen 9 im Ausgleichsvolumen 3 und erhöht sich das Volumen des Gasanteils 19, sodass der Druck im Ausgleichsvolumen 3 sinkt.

Ist der Volumeninhalt des Ausgleichsvolumens 3 genau bekannt, so kann im Prinzip aus einer solchen Druckänderung die aus dem Ausgleichsvolumen abgeflossene Flüssigkeitsmenge bestimmt und mit der durch den Flusssensor 2 gemessenen Flüssigkeitsmenge verglichen werden, um diesen zu kalibrieren oder zu überwachen. Allerdings ist der Volumeninhalt des Ausgleichsvolumens 3 üblicherweise nicht exakt bekannt. Insbesondere kann sich dieser Volumeninhalt dabei aufgrund von Fertigungstoleranzen bei der Fertigung des flüssigkeitsführenden Bereiches 20 von Disposable zu Disposable unterscheiden. Weiterhin können das Ausgleichsvolumen 3 oder die übrigen flüssigkeitsführenden Bereiche auch zumindest teilweise flexibel sein, und daher ihren Volumeninhalt unter Druckänderungen verändern.

Daher wird bei dem in Fig. 1 gezeigten Ausführungsbeispiel das maschinenseitige Referenzvolumen 4 eingesetzt, welches starr ist und dessen Volumeninhalt Vref exakt bekannt ist. Erfindungsgemäß wird dabei die Fluidverbindung über das zwischen dem Ausgleichsvolumen 3 und dem Referenzvolumen 4 über das Ventil C2 zu zumindest einem Zeitpunkt des Verfahrens geöffnet, sodass eine aufgrund des Abfließens einer Flüssigkeitsmenge über den Flusssensor 2 erzeugte Volumenänderung des Gasanteils 19 im Ausgleichsvolumen 3 den Druck im Referenzvolumen 4 verändert. Über diese Druckänderung im Referenzvolumen und den exakt bekannten Volumeninhalt des Referenzvolumens kann dann gegebenenfalls unter Verwendung weiterer Messwerte und insbesondere unter Verwendung mindestens eines absoluten Druckmesswertes im Referenzvolumen und / oder im Ausgleichsvolumen die Flüssigkeitsmenge, welche durch den Flusssensor-Abschnitt 2 geflossen ist, bestimmt und zur Kalibrierung und / oder Überwachung der durch den Flusssensor bestimmten Flüssigkeitsmenge herangezogen werden.

Im Hinblick auf die genaue Verfahrensführung ergeben sich dabei erfindungsgemäß mehrere Möglichkeiten, wie die durch den Flusssensor-Abschnitt 2 fließende Flüssigkeitsmenge anhand der im Ausgleichsvolumen 3 und / oder im Referenzvolumen 4 gemessenen Druckwerte bestimmt werden kann.

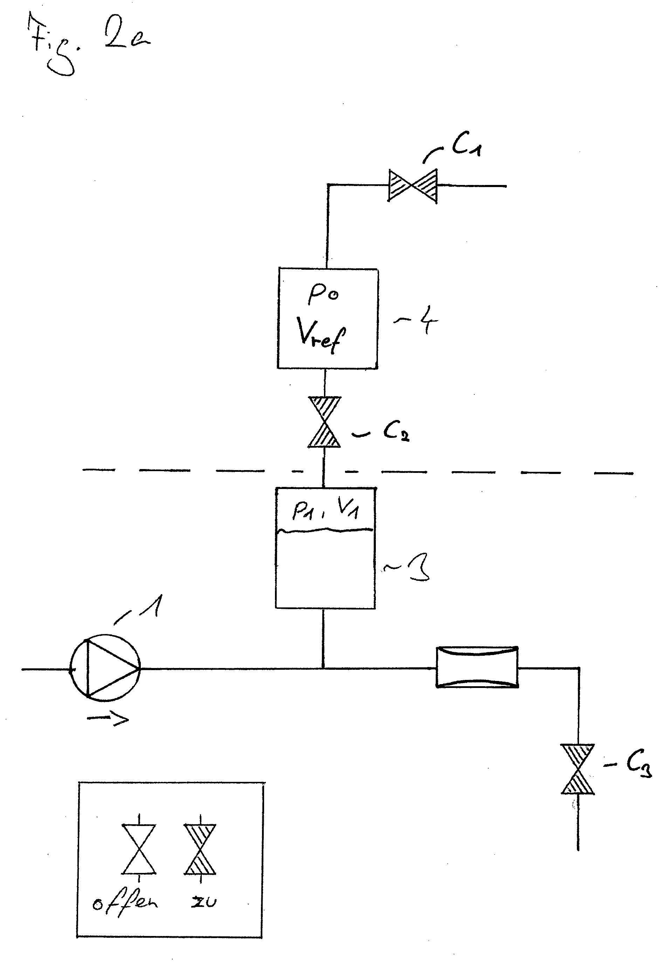

In Fig. 2a bis 2d sind dabei mehrere Schritte einer ersten Ausführungsform eines erfindungsgemäßen Verfahrens dargestellt. Dabei sind alle Ventile C1 bis C3 zum Startzeitpunkt offen, die Pumpe steht, und es herrscht im gesamten System Atmosphärendruck p0.

Bei dem in Fig. 2a gezeigten ersten Schritt werden dann alle Ventile C1 bis C3 geschlossen. Daraufhin wird die Pumpe 1 betätigt, um Flüssigkeit in das Ausgleichsvolumen 3 zu pumpen. Hierdurch stellt sich im Ausgleichsvolumen 3 ein Druck p1 ein, wobei der Gasanteil im Ausgleichsvolumen einen Volumeninhalt V1 aufweist. Im Referenzvolumen 4 herrscht dagegen weiterhin der Druck p0.

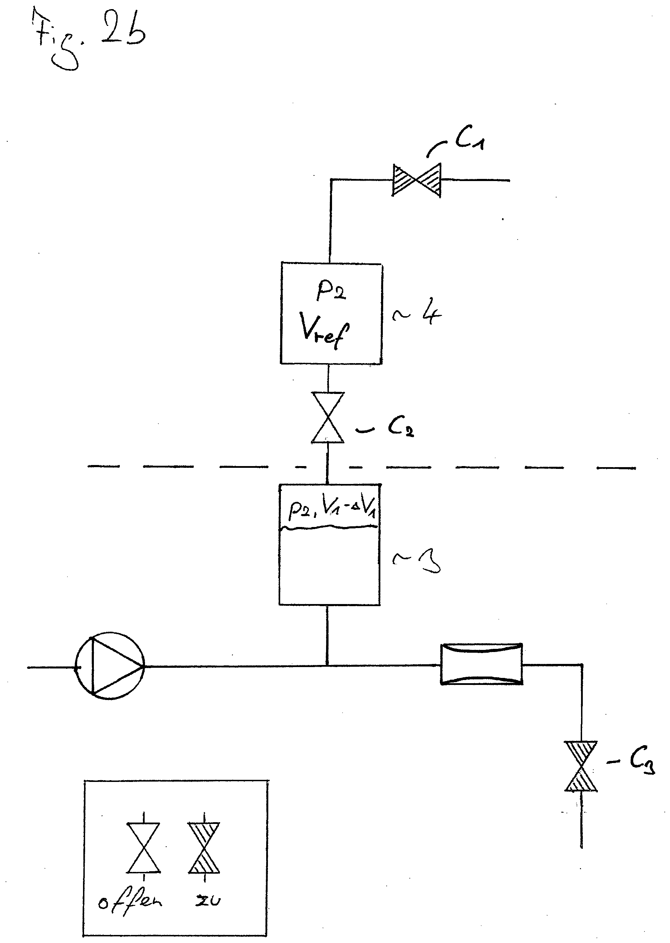

In dem in Fig. 2b gezeigten zweiten Schritt wird nun das Ventil C2 geöffnet. Hierdurch stellt sich im Ausgleichsvolumen 3 und im Referenzvolumen 4 der gleiche Druck p2 ein. Der Flüssigkeitspegel im Ausgleichsvolumen 3 kann sich dabei aufgrund einer etwaigen Incompliance des Disposables bei Druckänderung ebenfalls ändern, was zu einer Volumenänderung des Gasanteils ΔV1 führt. Bei rigiden Kassettensystemen besteht hier jedoch keine Relevanz, sodass der Term ΔV1 ignoriert werden kann. Ein Verfahren, welches den Term für nicht rigide Disposables wie beispielsweise Schlauchsysteme berücksichtigt, wird im Rahmen der zweiten Ausführungsform beschrieben.

Die Drücke p0, p1 und p2 werden gemessen, um aus dem bekannten Volumeninhalt Vref des Referenzvolumens 4 den Volumeninhalt V1 des Gasanteils im Ausgleichsvolumen 3 zu berechnen. Dabei gilt aufgrund der Gasgleichung:

$$p0 * Vref + p1 * V1 = p2 * Vref + p2 * V1$$

Hieraus folgt:

$$V1 = Vref * (p0 - p2) / (p2 - p1)$$

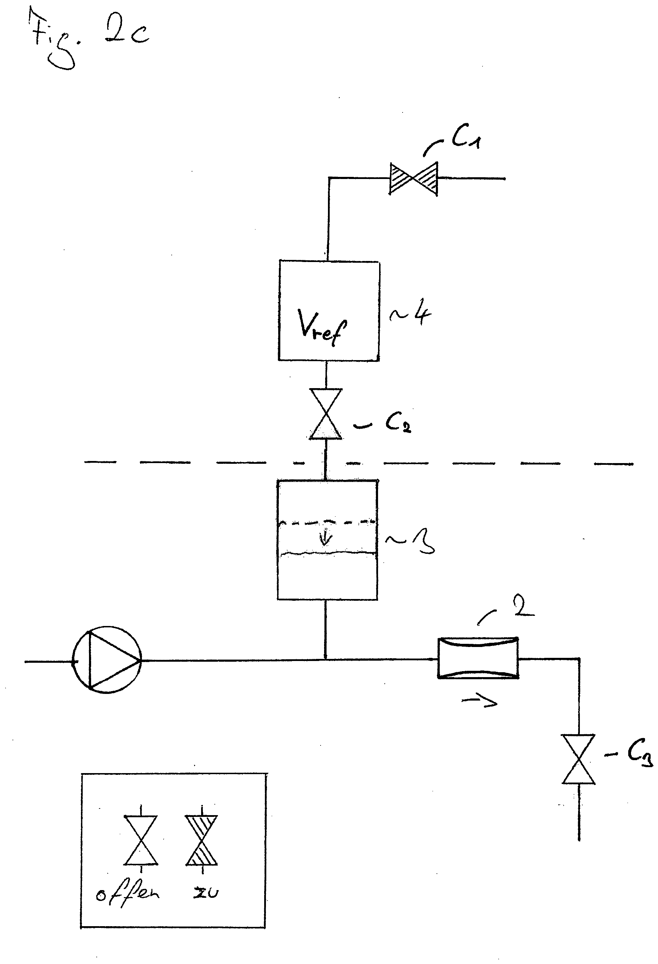

Bei dem in Fig. 2c gezeigten dritten Schritt wird nun das Ventil bzw. die Klemme C3 geöffnet, sodass Flüssigkeit über den Flüssigkeitssensor-Abschnitt 2 aus dem Ausgleichsvolumen 3 abfließt. Hierdurch sinkt der Flüssigkeitspegel im Ausgleichsvolumen 3 und es steigt der Volumeninhalt des Gasanteils im Ausgleichsvolumen. Dabei wird angenommen, dass der Systemdruck hinter C3 niedriger ist als der Druck p2.

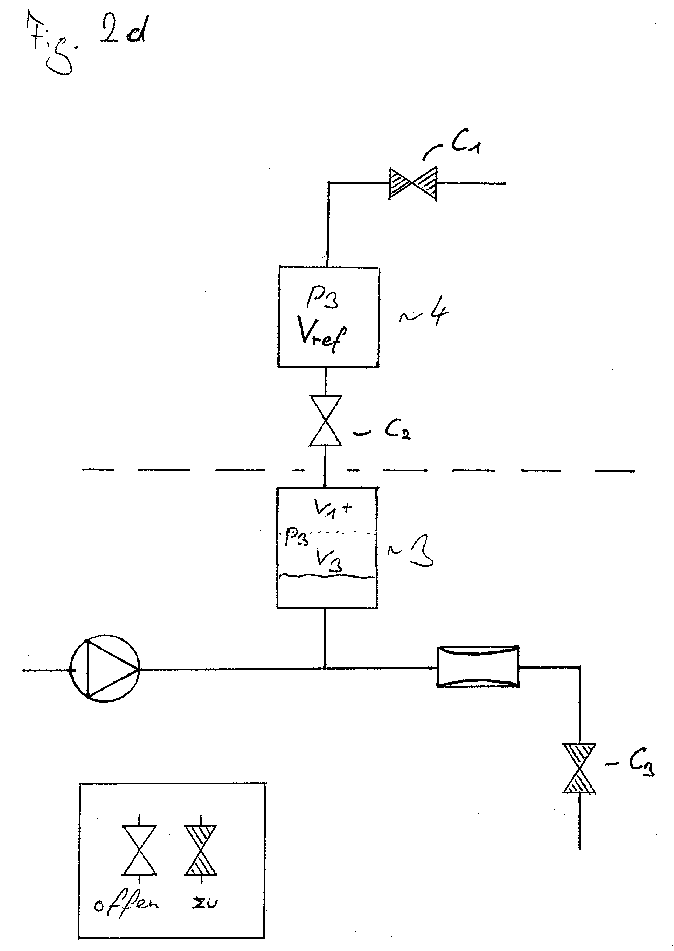

In Fig. 2d ist dabei die Endsituation dargestellt, in welcher eine Flüssigkeitsmenge V3 durch den Flusssensor-Abschnitt 2 abgeflossen ist, wodurch sich der Volumeninhalt des Gasanteils um V3 erhöht hat und sich im Ausgleichsvolumen und im Referenzvolumen ein Druck p3 eingestellt hat. In Fig. 2d ist dabei das Ventil bzw. die Klemme C3 als geschlossen dargestellt, sodass sich ein stationärer Druck einstellt. Folgen die Druckverhältnisse im Referenzvolumen und im Ausgleichsvolumen jedoch schnell genug den Flüssigkeitsständen, können die Druckwerte gegebenenfalls auch online gemessen und zur Berechnung heranzogen werden.

Der Druck p3 wird gemessen, um V3 zu berechnen. Dabei gilt aufgrund der Gasgesetze:

$$p2 * Vref + p2 * V1 = p3 * Vref + p3 * (V1 + V3)$$

Hierdurch ergibt sich für V3 folgender Wert:

$$V3 = Vref * (p2 - p3) / p3 * (1 + (p0 - p2) / (p2 - p1))$$

Mit der Kenntnis von V3 kann die im dritten Schritt durch den Flusssensor geflossene Volumenmenge absolut kalibriert werden. In gleicher Weise kann die Berechnung von V3 zur Überwachung des Flusssensors herangezogen werden.

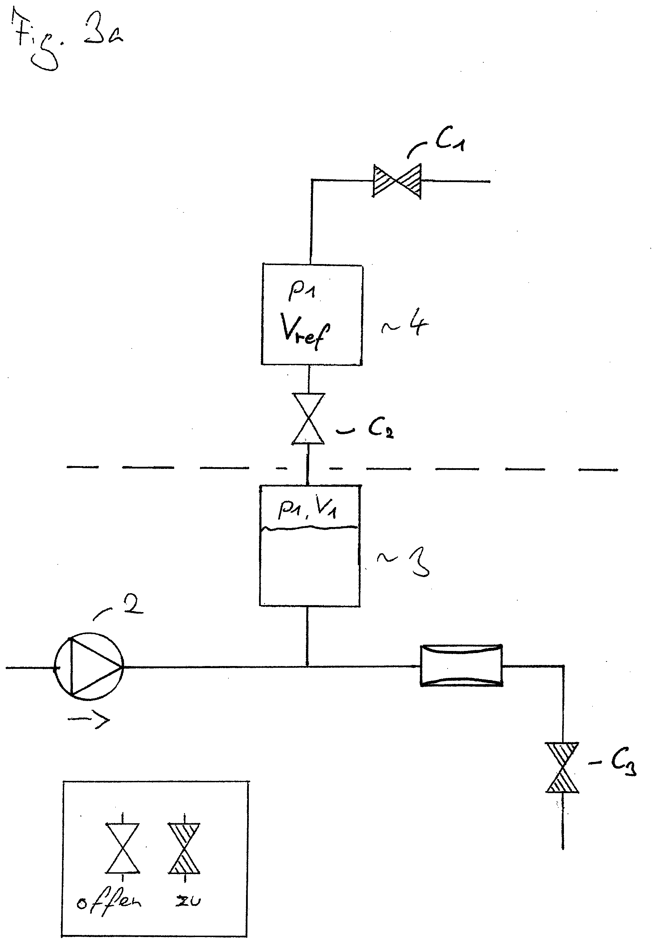

In Fig. 3a bis 3e ist nun ein zweites Ausführungsbeispiel eines erfindungsgemäßen Verfahrens dargestellt, welches auch bei nicht-rigiden Disposables und insbesondere bei Schlauchsets eingesetzt werden kann. Das Verfahren kann jedoch selbstverständlich auch bei starren Kassettensystemen eingesetzt werden.

Bei dem in Fig. 3a gezeigten ersten Schritt ist das Ventil C2 offen, während C1 und C3 geschlossen sind. Die Pumpe 1 fördert kurz Flüssigkeit und erzeugt damit einen Druck p1 im ganzen System vor C3. Der Gasanteil im Ausgleichsvolumen 3 hat dabei einen Volumeninhalt V1.

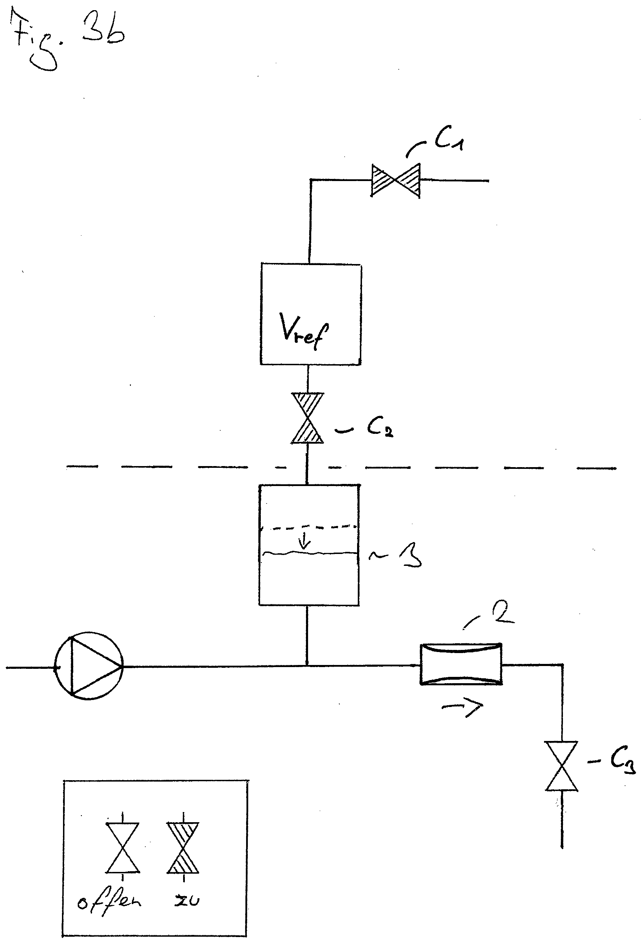

Nun wird in einem zweiten, in Fig. 3b gezeigten Schritt C2 geschlossen. Dann wird C3 geöffnet, so dass Flüssigkeit über den Flusssensor-Abschnitt 2 abfließt und sich der Flüssigkeitspegel im Ausgleichsvolumen 3 senkt.

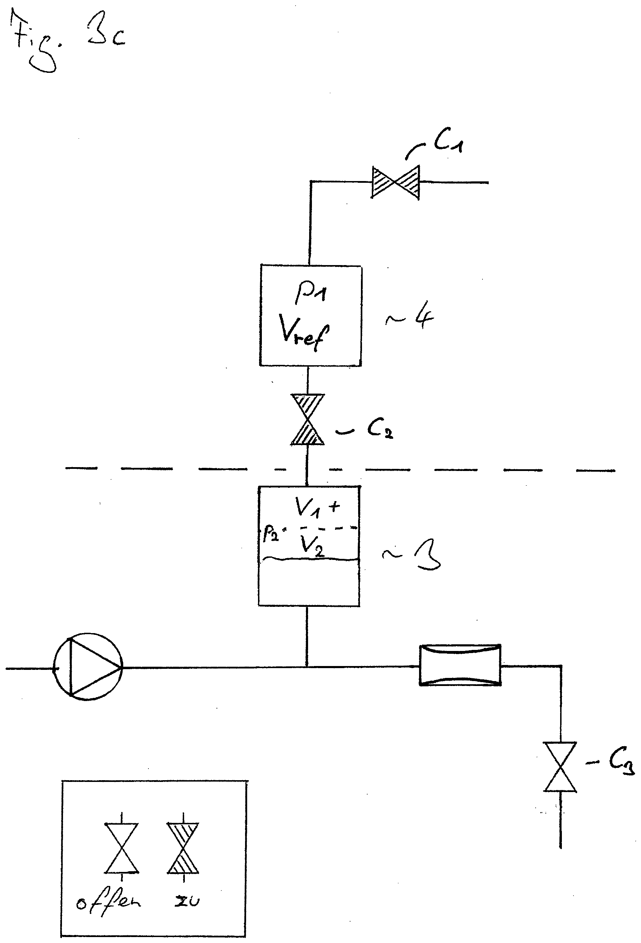

In Fig. 3c ist dabei ein Zustand dargestellt, in welchem im Ausgleichsvolumen 3 ein zusätzliches Gasvolumen V2 hinzugekommen ist, wodurch sich im Ausgleichsvolumen ein Druck p2 eingestellt hat. Im Referenzvolumen 4 herrscht dagegen aufgrund des geschlossenen Ventils C2 weiterhin der Druck p1. Vor der Messung von p2 kann dabei gegebenenfalls die Klemme bzw. Ventil C3 geschlossen werden, um einen stationären Zustand herzustellen.

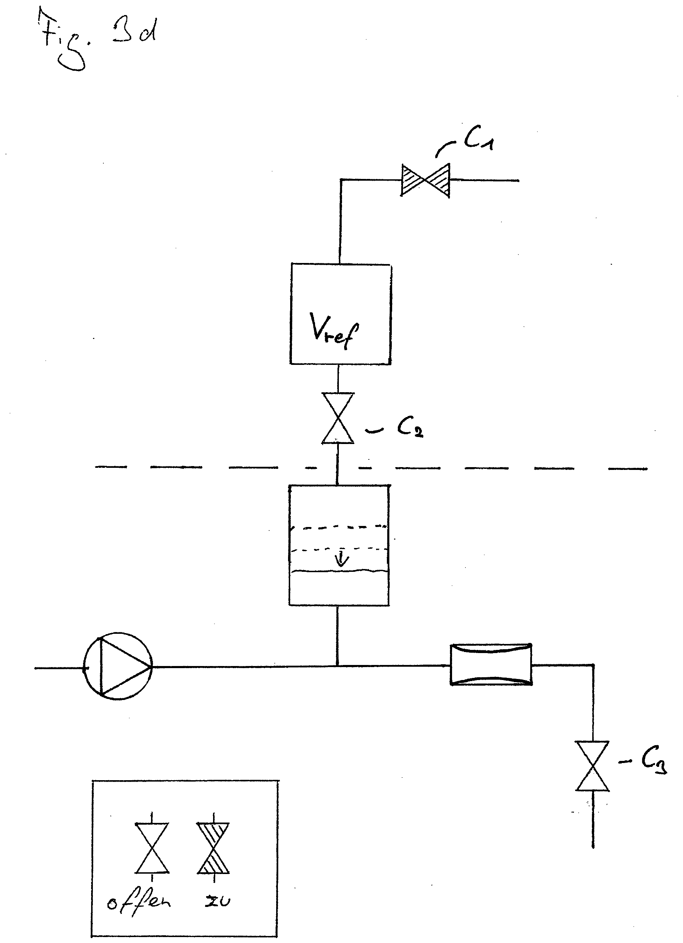

Bei dem in Fig. 3d dargestellten dritten Schritt wird nun C2 geöffnet, sodass ein Druckausgleich zwischen dem Ausgleichsvolumen 3 und dem Referenzvolumen 4 erfolgt und der Druck sich zunächst auf einen Mittelwert zwischen dem Druck p2 und dem Druck p1 einstellt. Das Ventil bzw. die Klemme C3 bleibt währenddessen entweder geöffnet, oder wird geöffnet, wobei der Druck im Ausgleichsvolumen bzw. im Referenzvolumen überwacht wird. C3 wird dabei geschlossen, sobald der Druck erneut p2 erreicht, um das weitere Ablaufen zu stoppen. Die hierbei durch den Sensor gelaufene Flüssigkeitsmenge V3 stellt das Kalibriervolumen dar.

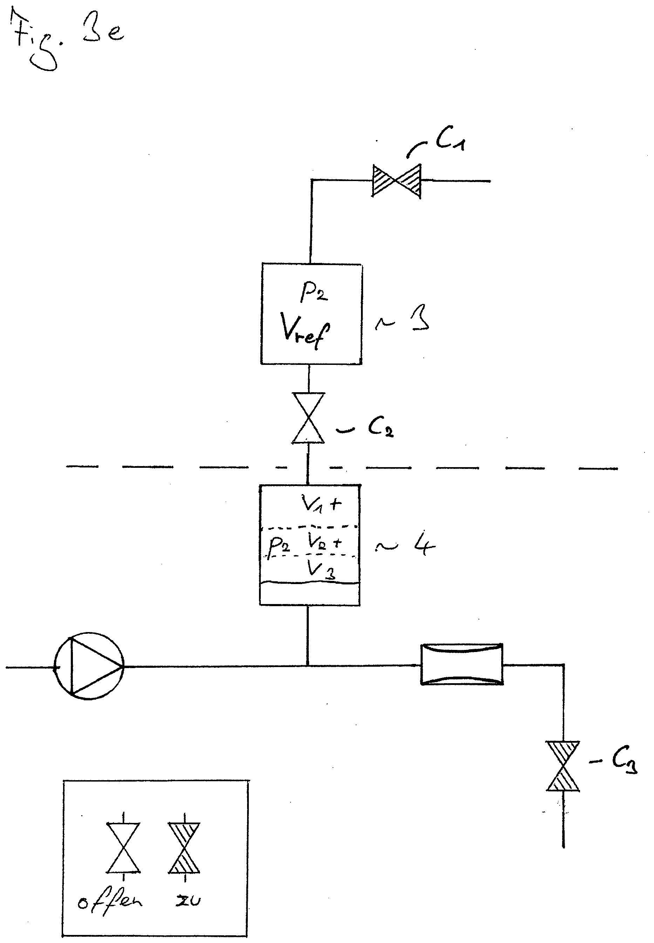

Die Endsituation ist dabei in Fig. 3e gezeigt: Der Gasanteile im Ausgleichsvolumen 4 weist einen Volumeninhalt von V1 + V2 + V3 auf, sowie durch das rechtzeitige Schließen der Klemme bzw. des Ventils C3 wieder den Druck p2. Der gleiche Druck p2 herrscht auch im Referenzvolumen 3. Bei der Referenz-Flüssigkeitsvolumenmenge V3 handelt es sich damit um die Flüssigkeitsmenge, welche nach dem Öffnen des Ventils C2 durch den Flusssensor 2 geflossen ist, bis der Druck im Ausgleichsvolumen 4 wieder das Druckniveau erreicht hat, welches er beim Öffnen des Ventils C2 hatte.

Aufgrund der gemessenen Drücke p1 und p2 sowie des bekannten Volumeninhalts Vref des Referenzvolumens 4 kann die Flüssigkeitsmenge V3 berechnet werden. Da der Druck p2 bei der Bestimmung von V2 und V3 gleich war, besteht Invarianz gegenüber einer möglichen Volumeninstabilität des Disposables bei verschiedenen Drücken und es gelten aufgrund des Gasgesetzes:

$$p1 * Vref + p2 * (V1 + V2) = p2 * Vref + p2 * (V1 + V2 + V3)$$

Hieraus folgt zunächst

$$p1 * Vref = p2 * Vref + p2 * V3,$$

und hieraus wiederum

$$V3 = Vref * (p1 - p2) / p2 .$$

Mit der Kenntnis von V3 kann die im dritten Schritt durch den Flusssensor geflossene Volumenmenge absolut kalibriert werden. In gleicher Weise kann die Berechnung von V3 zur Überwachung des Flusssensors herangezogen werden.

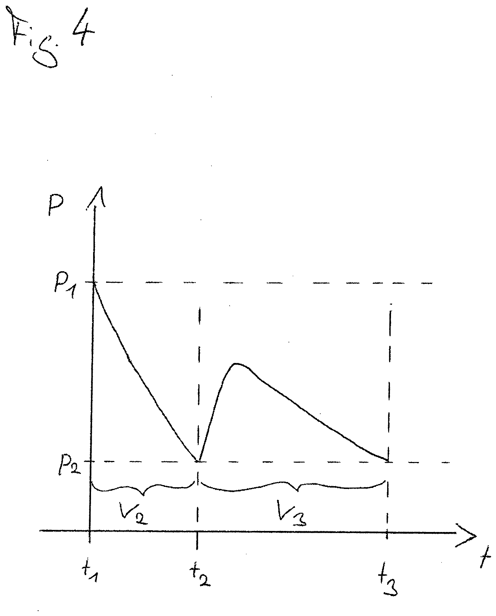

Der Druckverlauf im Ausgleichsvolumen 4 bei der Durchführung der zweiten Verfahrensvariante ist in Fig. 4 noch einmal in einer Prinzipdarstellung dargestellt. Der erste Schritt ist dabei zum Zeitpunkt t1 beendet, d. h. es herrscht im Ausgleichsvolumen der Druck p1. Daraufhin wird C3 geöffnet, sodass durch Abfließen des Volumens V2 sich der Druck auf p2 verringert. Zum Zeitpunkt t2 wird nun das Ventil C2 geöffnet, sodass der Druck durch einen Druckausgleich zwischen dem Ausgleichsvolumen und dem Referenzvolumen zunächst schnell ansteigt, und dann durch das Abfließen von Flüssigkeit über die noch bzw. wieder geöffnete Klemme bzw. das Ventil C3 wieder abfällt, bis der Druck p2 zum Zeitpunkt t3 wieder erreicht ist. Zum Zeitpunkt t3 wird die Klemme bzw. das Ventil C3 wieder geschlossen, bzw. die Bestimmung der Flüssigkeitsmenge V3 durch den Flusssensor 2 beendet. Die in den Phasen zwischen t1 und t2 bzw. zwischen t2 und t3 abfließenden Flüssigkeitsmengen V2 und V3 sind in Fig. 4 ebenfalls angegeben.

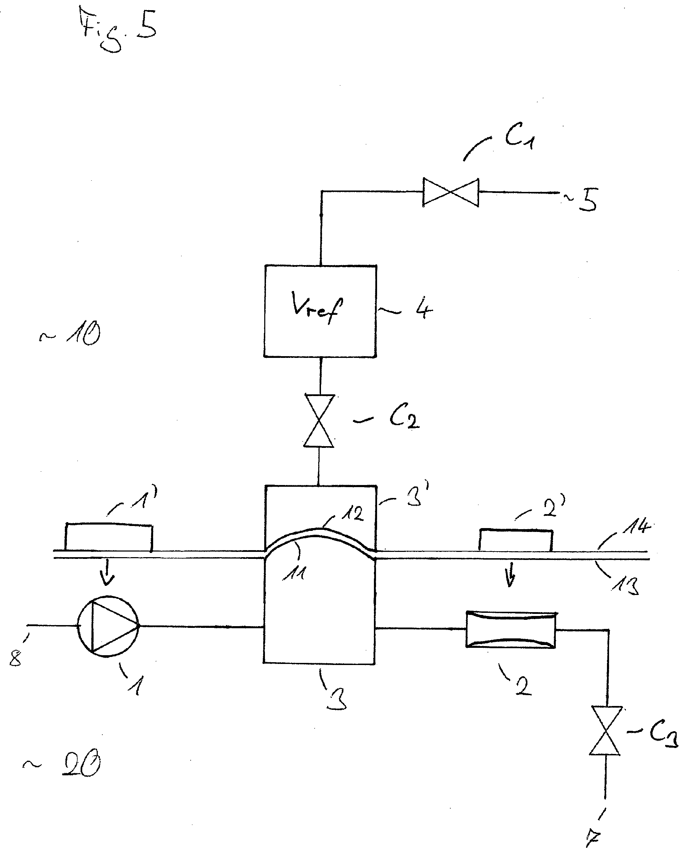

In Fig. 5 ist ein weiteres Ausführungsbeispiel eines erfindungsgemäßen Systems gezeigt, bei welchem die erfindungsgemäßen Verfahren zur Kalibrierung und Überwachung des Flusssensors ebenfalls eingesetzt werden können.

Das zweite Ausführungsbeispiel des erfindungsgemäßen Systems unterscheidet sich vom ersten Ausführungsbeispiel dadurch, dass das Ausgleichsvolumen nicht ausschließlich disposableseitig angeordnet ist, sondern auch auf der Maschinenseite ein Ausgleichsvolumen 3' vorgesehen ist. Das Ausgleichsvolumen 3 des Disposables ist dabei fluidisch von dem Ausgleichsvolumen 3' der Behandlungsmaschine 10 getrennt, jedoch mit diesem druckverbunden. Insbesondere sind die beiden Ausgleichsvolumina 3 und 3' dabei jeweils über eine Membran 11 bzw. 12 verschlossen, wobei die beiden Membranen 11 und 12 miteinander gekoppelt sind. Bei dem in Fig. 5 dargestellten Ausführungsbeispiel ist das Ausgleichsvolumen 3 des Disposables dabei als eine Kammer ausgeführt, welche in einer Ankoppelfläche 13 des Disposables angeordnet ist und über eine flexible Membran 11 im Bereich der Ankoppelfläche verschlossen wird. In gleicher Weise ist auch das Ausgleichsvolumen 3' der Behandlungsmaschine als eine Kammer ausgeführt, welche in der Ankoppelfläche 14 der Behandlungsmaschine eingelassen ist und über eine flexible Membran 12 im Bereich der Ankoppelfläche verschlossen ist. Die beiden Membranen liegen dabei aneinander auf, wobei bevorzugt zwischen den beiden Ankoppelflächen 13 und 14 ein Vakuum hergestellt wird, sodass die Bewegung der Membranen aneinander gekoppelt sind. Hierdurch herrschen in den beiden Ausgleichsvolumina 3 und 3' bis auf einen eventuell zu berücksichtigenden Einfluss der Eigenspannung der Membran die gleichen Drücke. Bei dem Disposable kann es sich dabei insbesondere um eine Kassette handeln.

Die erfindungsgemäßen Verfahrensvarianten können dabei mit dem zweiten Ausführungsbeispiel des erfindungsgemäßen Systems in gleicher Weise durchgeführt werden wie mit dem ersten Ausführungsbeispiel. Das disposableseitige Ausgleichsvolumen 3 ist hierfür entweder komplett oder teilweise mit Flüssigkeit gefüllt, das maschinenseitige Ausgleichsvolumen 3' ist dagegen komplett mit Gas. Eine Fluidverbindung wird über das Ventil C2 nur zwischen dem maschinenseitigen Ausgleichsvolumen 3' und dem Referenzvolumen 4 hergestellt. Dennoch besteht bei geöffnetem Ventil C2 eine Druckverbindung zwischen dem Referenzvolumen und dem disposableseitigen Ausgleichsvolumen 3. Änderungen des Flüssigkeitsstandes im Ausgleichsvolumen 3 des Disposables führen daher zu einer Druckänderung im maschinenseitigen Ausgleichsvolumen 3' und, wenn das Ventil C2 geöffnet ist, wie beim ersten Ausführungsbeispiel zu einer Druckänderung im Referenzvolumen 4.

In Fig. 5 sind weiterhin die in Fig. 1 nicht dargestellten maschinenseitigen Komponenten der Pumpe bzw. des Flusssensors schematisch dargestellt. Insbesondere kann dabei eine Rollenpumpe 1' vorgesehen sein, in welche der Pumpabschnitt 1 einlegbar ist, sowie die maschinenseitigen Flusssensorkomponenten 2', an welche der Flusssensorabschnitt 2 des Disposables gekoppelt wird. Weiterhin wird bevorzugt auch das Ventil bzw. die Klemme C3 über einen maschinenseitig angeordneten Ventil- und / oder Klemmenaktor betätigt, was in Fig. 5 jedoch ebenfalls nicht dargestellt ist.

Beiden Ausführungsbeispielen ist gemeinsam, dass es keine "nasse" Verbindung zwischen dem Disposable und der Behandlungsmaschine gibt. Im ersten Ausführungsbeispiel wird der Flüssigkeitsübertritt durch den Hydrophob-Filter verhindert, im zweiten Ausführungsbeispiel durch die Membran. Alternativ oder zusätzlich könnte ein Flüssigkeitsübertritt von dem Disposable zur Behandlungsmaschine auch durch eine entsprechende Verfahrensführung gewährleistet werden, insbesondere durch eine entsprechende Steuerung der auf beiden Seiten herrschenden Drücke und/oder durch eine Level-Detektion auf Seiten des Disposables.

Die vorliegende Erfindung erlaubt unabhängig von der konkreten Ausführungsform eine Absolutkalibrierung und/oder Überwachung eines Flusssensors durch die Behandlungsmaschine und insbesondere das Dialysesystem. Dabei wird das Ausgleichsvolumen mit Druck beaufschlagt. Daraufhin wird eine Fluidverbindung geöffnet, sodass Flüssigkeit aus dem Ausgleichsvolumen durch den Flusssensor fließt. Aus der Druckänderung im Ausgleichsvolumen (und/oder dem damit verbundenen Refenzvolumen) wird die Flüssigkeitsmenge berechnet, die durch den Flusssensor geflossen ist. Hierdurch kann ein einfacher Flusssensor eingesetzt werden, und dementsprechend Kosten, Gewicht und Bauraum gespart werden. Dennoch ist eine exakte und einfache Kalibrierung und Überwachung möglich.

Die Behandlungsmaschine weist dabei bevorzugt eine Steuerung auf, welche die Kalibrierung und / oder Überwachung automatisch durchführt und insbesondere mindestens eines der oben näher beschriebenen Verfahren automatisch durchführt.

Die Kalibrierung kann dabei jeweils zu Beginn einer Behandlung im Rahmen eines Initialtests erfolgen. Weiterhin bevorzugt erfolgt die Überwachung in regelmäßigen Abständen während der Behandlung. Insbesondere kann dabei in regelmäßigen Abständen einer normalen Pumpsequenz eine erfindungsgemäße Verfahrenssequenz zwischengeschaltet werden, um die Genauigkeit des Flusssensors zu überwachen und diesen gegebenenfalls nachzukalibrieren. Auch dies kann automatisch über die Steuerung der Behandlungsmaschine durchgeführt werden.

**Patentansprüche**

1. Verfahren zur Kalibrierung und/oder Überwachung eines Fluss-Sensors eines medizinischen Systems, welcher mit einem Ausgleichsvolumen fluidisch verbunden und/oder verbindbar ist, mit den Schritten:

   - Einstellen oder Messen eines ersten Druckniveaus (P1),
   - Öffnung einer Fluidverbindung (C3), so dass Flüssigkeit durch den Fluss-Sensor (2) aus dem oder in das Ausgleichsvolumen fließt,
   - Messen einer durch den Flüssigkeitssensor fließenden ersten Flüssigkeitsmenge V3 mittels des Fluss-Sensors,
   - Einstellen oder Messen eines zweiten Druckniveaus (P2),
   - Bestimmen der ersten Flüssigkeitsmenge V3 anhand des ersten und des zweiten Druckniveaus und
   - Kalibrierung und/oder Überwachung des Fluss-Sensors anhand der mittels des Fluss-Sensors gemessenen ersten Flüssigkeitsmenge und der anhand des ersten und des zweiten Druckniveaus bestimmten ersten Flüssigkeitsmenge,

   **dadurch gekennzeichnet,**
   **dass** eine Beaufschlagung des Ausgleichvolumens mit Druck mittels einer Pumpe des medizinischen Systems erfolgt, welche vorhanden ist, um eine medizinische Flüssigkeit innerhalb des medizinischen Systems zu bewegen.

**2.** Verfahren nach Anspruch 1, wobei ein mit dem Ausgleichvolumen gekoppeltes starres Referenzvolumen eingesetzt wird und die Bestimmung der Flüssigkeitsmenge anhand des bekannten Volumeninhalts Vref des Referenzvolumens und mindestens einer Druckmessung im Referenzvolumen erfolgt, wobei das Referenzvolumen bevorzugt mit Gas gefüllt ist.

**3.** Verfahren nach Anspruch 2, wobei eine Druckverbindung zwischen dem Ausgleichvolumen und dem Referenzvolumen mindestens einmal geschlossen und/oder geöffnet wird, wobei die Druckverbindung zwischen dem Ausgleichsvolumen und dem Referenzvolumen bevorzugt geöffnet ist oder wird, während oder nachdem Flüssigkeit durch den Fluss-Sensor aus dem oder in das Ausgleichsvolumen fließt, und/oder wobei der Druck im Ausgleichvolumen und/oder im Referenzvolumen bevorzugt vor und nach dem Öffnen und/oder Schließen der Druckverbindung bestimmt wird.

**4.** Verfahren nach einem der vorangegangenen Ansprüche, wobei der Druck im Ausgleichvolumen und/oder im Referenzvolumen vor und nach dem Abfließen der ersten Flüssigkeitsmenge V3 in das oder aus dem Ausgleichsvolumen bestimmt wird und/oder wobei die erste Flüssigkeitsmenge V3 anhand der Druckänderung im Ausgleichvolumen und/oder im Referenzvolumen bestimmt wird, und/oder wobei die erste Flüssigkeitsmenge V3 anhand einer Druckänderung und mindestens einem absoluten Druckniveau bestimmt wird.

**5.** Verfahren nach einem der vorangegangenen Ansprüche, wobei eine Beaufschlagung des Ausgleichvolumens mit Druck mittels einer Pumpe des medizinischen Systems erfolgt, welche die Flüssigkeit im Normalbetrieb des Systems durch den Fluss-Sensor transportiert, sodass der Fluss-Sensor zum Messen der durch die Pumpe gepumpten Flüssigkeit eingesetzt werden kann, wobei bevorzugt zur Beaufschlagung des Ausgleichsvolumens mit Druck eine Fluidverbindung geschlossen wird, sodass die von der Pumpe in das Ausgleichsvolumen gepumpte Flüssigkeit nicht wie üblich abfließen kann und daher den Druck im Ausgleichsvolumen erhöht, und/oder wobei die Pumpe über eine Fluidverbindung mit dem Ausgleichsvolumen verbunden oder verbindbar ist und/oder wobei es sich bei der Pumpe um eine okkludierende Pumpe, insbesondere eine Rollenpumpe handelt.

**6.** Verfahren nach einem der Ansprüche 2 bis 5, wobei in einem ersten Schritt ein Druck p1 in dem Ausgleichsvolumen erzeugt wird, während die Druckverbindung zum Referenzvolumen getrennt ist und dort ein Druck p0 gemessen wird, und/oder wobei in einem zweiten Schritt die Druckverbindung zwischen dem Ausgleichsvolumen und dem Referenzvolumen geöffnet wird, so dass sich in dem Ausgleichsvolumen und dem Referenzvolumen ein gemeinsamer Druck p2 einstellt, und/oder wobei in einem dritten Schritt die Fluidverbindung geöffnet wird, so dass Flüssigkeit durch den Fluss-Sensor aus dem oder in das Ausgleichsvolumen fließt, wobei die dabei durch den Flüssigkeitssensor fließende erste Flüssigkeitsmenge V3 mittels des Fluss-Sensors gemessen wird und der sich durch das Zu- oder Abfließen der ersten Flüssigkeitsmenge V3 in dem Ausgleichvolumen und dem Referenzvolumen einstellende Druck p3 gemessen wird.

**7.** Verfahren nach Anspruch 6, wobei die erste Flüssigkeitsmenge V3 aus den Drücken p0, p1, p2 und p3 und dem Volumeninhalt Vref des Referenzvolumens bestimmt wird, wobei bevorzugt gilt:

$$V3 = Vref * (p2 - p3) / p3 * (1 + (p0 - p2) / (p2 - p1))$$

**8.** Verfahren nach einem der Ansprüche 2 bis 5, wobei die Fluidverbindung solange geöffnet wird, bis sich in dem Ausgleichvolumen durch Zu- oder Abfließen einer Flüssigkeitsmenge ein bestimmter Druck eingestellt hat, und/oder wobei in einem ersten Schritt ein Druck p1 in dem Ausgleichsvolumen erzeugt wird, während die Druckverbindung zum Referenzvolumen geöffnet ist, und/oder wobei in einem zweiten Schritt die Druckverbindung zwischen dem Ausgleichsvolumen und dem Referenzvolumen geschlossen und die Fluidverbindung geöffnet wird, so dass eine zweite Flüssigkeitsmenge V2 durch den Fluss-Sensor aus dem oder in das Ausgleichsvolumen fließt, wobei der sich durch das Zu- oder Abfließen der zweiten Flüssigkeitsmenge V2 in dem Ausgleichsvolumen einstellende Druck p2 gemessen wird, und/oder wobei in einem dritten Schritt die Druckverbindung zwischen dem Ausgleichsvolumen und dem Referenzvolumen geöffnet wird und die Fluidverbindung solange geöffnet wird, bis sich in dem Ausgleichvolumen und dem Referenzvolumen durch Abfließen der ersten Flüssigkeitsmenge V3 wieder der Druck p2 eingestellt hat.

**9.** Verfahren nach Anspruch 8, wobei die erste Flüssigkeitsmenge V3 aus den Drücken p1 und p2 und dem Volumeninhalt Vref des Referenzvolumens bestimmt wird, wobei bevorzugt gilt:

$$V3 = Vref * (p1 - p2) / p2$$

**10.** Medizinisches System mit einer Behandlungsmaschine (10) und einem Disposable (20) mit flüssigkeitsführenden Bereichen, welches an die Behandlungsmaschine ankoppelbar ist, wobei das Disposable einen Fluss-Sensor-Abschnitt (2) aufweist, und wobei das Disposable weiterhin ein Ausgleichsvolumen (3) aufweist, welches mit dem Fluss-Sensor-Abschnitt fluidisch verbunden und/oder verbindbar ist, wobei das Ausgleichsvolumen an ein Referenz-Volumen (4) der Behandlungsmaschine ankoppelbar ist oder wobei das Disposable ein Referenz-Volumen aufweist, welches mit dem Ausgleichsvolumen über eine Druckverbindung verbunden oder verbindbar ist, **dadurch gekennzeichnet, dass** das medizinische System eine Pumpe umfasst, welche vorhanden ist, um eine medizinische Flüssigkeit innerhalb des medizinischen Systems zu bewegen, wobei mittels der Pumpe das Ausgleichvolumens mit Druck beaufschlagbar ist.

**11.** Medizinisches System nach Anspruch 10 mit einem Pump-Abschnitt (1), welcher mit dem Ausgleichsvolumen fluidisch verbunden oder verbindbar ist, wobei es sich bei dem Pump-Abschnitt bevorzugt um einen in eine okkludierende Pumpe der Behandlungsmaschine einlegbaren Schlauch-Abschnitt handelt.

**12.** Medizinisches System nach Anspruch 10 oder 11, wobei die Behandlungsmaschine mindestens einen und bevorzugt mindestens zwei Drucksensoren aufweist, über den oder die der Druck im Ausgleichsvolumen und/oder im Referenzvolumen messbar ist, und/oder wobei die Behandlungsmaschine mindestens einen Ventil- und/oder Klemmenaktor (C3) aufweist, über welchen eine Fluidverbindung im Disposable, über welche Flüssigkeit durch den Fluss-Sensor aus dem oder in das Ausgleichsvolumen fließen kann, geschlossen und geöffnet werden kann, und/oder wobei die Behandlungsmaschine mindestens einen Ventil- und/oder Klemmenaktor aufweist, über welchen eine Druckverbindung zwischen dem Ausgleichsvolumen und dem Referenzvolumen geöffnet und geschlossen werden kann, und/oder wobei die Behandlungsmaschine mindestens einen Ventil- und/oder Klemmenaktor (C2) aufweist, über welchen eine Druckverbindung zwischen dem Referenzvolumen und einem Druckbereich, insbesondere der Atmosphäre, geöffnet und geschlossen werden kann, und/oder wobei die Behandlungsmaschine mindestens einen Fluss-Sensor aufweist, welcher mit dem Fluss-Sensor-Abschnitt des Disposables koppelbar ist, wobei es sich bevorzugt um einen Ultraschall-Fluss-Sensor oder eine Differenzdrucksensor oder einen magnetisch-induktiven Sensor handelt.

**13.** Medizinisches System, insbesondere medizinisches System nach einem der Ansprüche 10 bis 12, mit einer Steuerung zur Auswertung der Signale eines Fluss-Sensors, wobei die Steuerung eine Kalibrierungs- und/oder Überwachungsfunktion zur Kalibrierung und/oder Überwachung des Fluss-Sensors nach einem Verfahren nach einem der Ansprüche 1 bis 9 aufweist, wobei die Kalibrierung und/oder Überwachung bevorzugt automatisch von der Steuerung vorgenommen wird und/oder wobei das Verfahren bevorzugt automatisch von der Steuerung durchgeführt wird.

**14.** Disposable eines medizinischen Systems nach einem der Ansprüche 10 bis 12.

**15.** Behandlungsmaschine eines medizinischen Systems nach einem der Ansprüche 10 bis 12 oder nach Anspruch 13 wenn abhängig von einem der Ansprüche 10-12.

**Claims**

**1.** A method for the calibration and/or monitoring of a flow sensor of a medical system, which is fluidly connected and/or connectable with a compensating volume, with the following steps:

- Setting or measuring a first pressure level (P1),
- Opening a fluid connection (C3), so that fluid flows through the flow sensor (2) out of or into the compensating volume,
- Measuring a first liquid quantity, V3, flowing through the liquid sensor, by means of the flow sensor,
- Setting or measuring a second pressure level (P2),

- Determining the first liquid quantity, V3, by means of the first and second pressure levels, and
- Calibration and/or monitoring of the flow sensor by means of the first liquid quantity measured by means of the flow sensor and the first liquid quantity determined by means of the first and of the second pressure levels, **characterized in that**

an application of the compensation volume with pressure occurs by means of a pump of the medical system, which is present to move a medical liquid within the medical system.

**2.** The method according to Claim 1, wherein a rigid reference volume coupled with the compensating volume is used and the determination of the liquid quantity results by means of the known volumetric content, Vref, of the reference volume and at least one pressure measurement in the reference volume, wherein the reference volume is preferably filled with gas.

**3.** The method according to Claim 2, wherein a pressure connection between the compensating volume and the reference volume is closed and/or opened at least once, wherein the pressure connection between the compensating volume and the reference volume preferably has been or is opened, during or after fluid flows through the flow sensor out of or into the compensating volume, and/or wherein the pressure in the compensating volume and/or reference volume is determined, preferably before and after opening and/or closing the pressure connection.

**4.** The method according to one of the preceding Claims, wherein the pressure in the compensating volume and/or reference volume is determined before and after the flow of the first liquid quantity V3 into or out of the compensating volume and/or wherein the first liquid quantity V3 is determined by means of the pressure change in the compensating volume and/or reference volume, and/or wherein the first liquid quantity V3 is determined by means of a pressure change and at least one absolute pressure level.

**5.** The method according to one of the preceding Claims, wherein an application of the compensating volumes with pressure occurs by means of a medical system pump, which, in normal operation of the system, moves the fluid through the flow sensor, so that the flow sensor for measuring the liquid pumped through the pump can be used, wherein preferably for application of the compensating volume with pressure, a fluid connection is closed, so that the liquid pumped into the compensation volume by the pump can not drain as usually and therefore increases the pressure in the compensation volume, and/or wherein the pump is connected or connectable to the compensation volume via a fluid connection and/or wherein the pump is an occluding pump, in particular a roller pump.

**6.** The method according to one of Claims 2 - 5, wherein in a first step, a pressure p1 is generated in the compensation volume, while the pressure connection to the reference volume is disconnected and a pressure p0 is measured there, and/or wherein in a second step, the pressure connection between the compensation volume and the reference volume is opened, so that a common pressure p2 forms in the compensation volume and the reference volume, and/or wherein in a third step, the fluid connection is opened, so that fluid flows through the flow sensor out of or into the compensating volume, wherein the first liquid quantity, V3, flowing thereby through the liquid sensor, is measured by means of the flow sensor and pressure p3 established through the in- or outflow of the first liquid quantity, V3, in the compensating volume and the reference volume is measured.

**7.** The method according to Claim 6, wherein the first liquid quantity, V3, is determined from the pressures p0, p1, p2 and p3 and the volumetric content Vref of the reference volume, wherein preferably the following applies:

$$V3 = Vref * (p2 - p3) / p3 * (1 + (p0 - p2) / (p2 - p1)).$$

**8.** The method according to one of Claims 2 - 5, wherein the fluid connection is opened long enough to set a determined pressure in the compensating volume through in- or outflow of a liquid quantity, and/or wherein in a first step, a pressure p1 is generated in the compensating volume, while the pressure connection to the reference volume is opened, and/or wherein, in a second step, the pressure connection between the compensating volume and the reference volume is closed and the fluid connection is opened, so that a second liquid quantity, V2, flows through the flow sensor out of the or into the compensating volume, wherein the p2 pressure set through the in- or outflow the second liquid quantity, V2, in the compensating volume is measured, and/or wherein, in a third step, the pressure connection between the compensating volume and the reference volume is opened and the fluid connection opened long enough to set the p2 pressure again in the compensating volume and the reference volume through the outflow of the first V3 liquid quantity.

9. The method according to claim 8, wherein the first liquid quantity V3 is determined from the pressures p1 and p2 and the volumetric content Vref of the reference volume, preferably wherein:

$$V3 = Vref * (p1 - p2) / p2.$$

10. A medical system with a treatment machine (10) and a disposable (20) with fluid distribution areas, which can be coupled with the treatment machine, wherein the disposable comprises a flow sensor section (2), and wherein the disposable further comprises a compensating volume (3), which is fluidly connected and/or connectable with the flow sensor section, wherein the compensating volume can be coupled with a reference volume (4) of the treatment machine or wherein the disposable comprises a reference volume, which is connected or connectable with the compensating volume via a pressure connection,
**characterized in that**
the medical system includes a pump, which is present for moving a medical liquid within the medical system, wherein the compensation volume can be applied with pressure by means of the pump.

11. The medical system according to claim 10, with a pump section (1), which is fluidly connected or connectable with the compensating volume, wherein the pump section is preferably a hose section, insertable in an occluding pump of the treatment machine.

12. The medical system according to Claim 10 or 11, wherein the treatment machine comprises at least one, and preferably at least two pressure sensors via which the pressure in the compensating volume and/or in reference volume can be measured, and/or wherein the treatment machine comprises at least one valve and/or clamp actuator (C3), via which a fluid connection in the disposable can be closed and opened, via which fluid can flow through the flow sensor out of or into in the compensating volume, and/or wherein the treatment machine comprises at least one valve and/or clamp actuator, via which a pressure connection between the compensating volume and the reference volume can be opened and closed, and/or wherein the treatment machine comprises at least one valve and/or clamp actuator (C2), via which a pressure connection can be opened and closed between the reference volume and a pressure range, in particular the atmosphere, and/or wherein the treatment machine comprises at least one flow sensor, which can be coupled with the flow sensor section of the disposable, wherein it is preferably an ultrasound flow sensor or a differential pressure sensor or a magnetic-inductive sensor.

13. The medical system, in particular a medical system according to one of Claims 10 to 12, with a control for evaluating the signals from a flow sensor, wherein the control comprises a calibration and/or monitoring function for the calibration and/or monitoring of the flow sensor according to a method pursuant to one of Claims 1 to 9, wherein the calibration and/or monitoring is preferably performed automatically by the control and/or wherein the method is preferably performed automatically by the control.

14. A disposable of a medical system according to one of Claims 10 to 12.

15. A treatment machine of a medical system according to one of Claims 10 to 12 or according to Claim 13, if dependent on one of claims 10 to 12.

**Revendications**

1. Procédé servant à l'étalonnage et/ou à la surveillance d'un capteur de flux d'un système médical, lequel est relié et/ou peut être relié de manière fluidique à un volume de compensation, avec les étapes :

    - de réglage ou de mesure d'un premier niveau de pression (P1),
    - d'ouverture d'une liaison fluidique (C3) de sorte que du liquide s'écoule à travers le capteur de flux (2) hors du ou dans le volume de compensation,
    - de mesure d'une première quantité de liquide V3 s'écoulant à travers le capteur de liquide au moyen du capteur de flux,
    - de réglage ou de mesure d'un deuxième niveau de pression (P2),
    - de définition de la première quantité de liquide V3 à l'aide du premier et du deuxième niveau de pression, et
    - d'étalonnage et/ou de surveillance du capteur de flux à l'aide de la première quantité de liquide mesurée au moyen du capteur de flux et de la première quantité de liquide définie à l'aide du premier et du deuxième niveau de pression,

**caractérisé en ce**

**que** une sollicitation du volume de compensation s'effectue avec une pression au moyen d'une pompe du système médical, qui est présente pour déplacer un liquide médical à l'intérieur du système médical.

2. Procédé selon la revendication 1, dans lequel un volume de référence rigide couplé au volume de compensation est utilisé et la définition de la quantité de liquide est effectuée à l'aide du contenu de volume Vref connu du volume de référence et au moins d'une mesure de pression dans le volume de référence, dans lequel le volume de référence est rempli de manière préférée de gaz.

3. Procédé selon la revendication 2, dans lequel une liaison sous pression entre le volume de compensation et le volume de référence est fermée et/ou ouverte au moins une fois, dans lequel la liaison sous pression est ou soit de manière préférée ouverte entre le volume de compensation et le volume de référence, pendant ou après que du liquide s'écoule à travers le capteur de flux hors du ou dans le volume de compensation, et/ou dans lequel la pression dans le volume de compensation et/ou dans le volume de référence est définie de manière préférée avant et après l'ouverture et/ou la fermeture de la liaison sous pression.

4. Procédé selon l'une quelconque des revendications précédentes, dans lequel la pression dans le volume de compensation et/ou dans le volume de référence est définie avant et après l'écoulement sortant de la première quantité de liquide V3 dans le ou hors du volume de compensation et/ou dans lequel la première quantité de liquide V3 est définie à l'aide de la modification de pression dans le volume de compensation et/ou dans le volume de référence, et/ou dans lequel la première quantité de liquide V3 est définie à l'aide d'une modification de pression et d'au moins un niveau de pression absolu.

5. Procédé selon l'une quelconque des revendications précédentes, dans lequel le volume de compensation est soumis à l'action d'une pression au moyen d'une pompe du système médical, laquelle pompe transporte le liquide dans le mode de fonctionnement normal du système à travers le capteur de flux de sorte que le capteur de flux peut être utilisé pour mesurer le liquide pompé par la pompe, dans lequel une liaison fluidique est fermée de manière préférée pour soumettre le volume de compensation à l'action d'une pression de sorte que le liquide pompé par la pompe dans le volume de compensation ne peut s'écouler comme d'habitude et par voie de conséquence augmente la pression dans le volume de compensation, et/ou dans lequel la pompe est reliée ou peut être reliée par l'intermédiaire d'une liaison fluidique au volume de compensation et/ou dans lequel la pompe est une pompe occlusive, en particulier une pompe à rouleaux.

6. Procédé selon l'une quelconque des revendications 2 à 5, dans lequel lors d'une première étape, une pression p1 est générée dans le volume de compensation, pendant la liaison sous pression est séparée par rapport au volume de référence et une pression p0 y est mesurée et/ou dans lequel lors d'une deuxième étape, la liaison sous pression est ouverte entre le volume de compensation et le volume de référence de sorte qu'une pression commune p2 s'établit dans le volume de compensation et dans le volume de référence, et/ou dans lequel lors d'une troisième étape, la liaison fluidique est ouverte de sorte que du liquide s'écoule à travers le capteur de flux hors du ou dans le volume de compensation, dans lequel la première quantité de liquide V3 s'écoulant ce faisant à travers le capteur de liquide est mesurée au moyen du capteur de flux et la pression s'établissant du fait de l'écoulement entrant et de l'écoulement sortant de la première quantité de liquide V3 dans le volume de compensation et le volume de référence est mesurée.

7. Procédé selon la revendication 6, dans lequel la première quantité de liquide V3 est définie à partir des pressions p0, p1, p2 et p3 et du contenu de volume Vref du volume de référence, dans lequel s'applique de manière préférée :

$$V3 = Vref * (p2 - p3) / p3 * (1 + (p0 - p2) / (p2 - p1)).$$

8. Procédé selon l'une quelconque des revendications 2 à 5, dans lequel la liaison fluidique est ouverte jusqu'à ce qu'une certaine pression se soit établie dans le volume de compensation du fait de l'écoulement entrant ou de l'écoulement sortant d'une quantité de liquide, et/ou dans lequel lors d'une première étape, une pression p1 est générée dans le volume de compensation pendant que la liaison sous pression avec le volume de référence est ouverte et/ou dans lequel lors d'une deuxième étape, la liaison sous pression entre le volume de compensation et le volume de référence est fermée et la liaison fluidique est ouverte de sorte qu'une deuxième quantité de liquide V2 s'écoule à travers le capteur de flux hors du ou dans le volume de compensation, dans lequel la pression p2 s'établissant du fait de l'écoulement entrant ou de l'écoulement sortant de la deuxième quantité de liquide V2 dans le volume de compensation est mesurée, et/ou dans lequel lors d'une troisième étape, la liaison sous pression entre le volume de compensation et le volume de référence est ouverte et la liaison fluidique est ouverte jusqu'à ce que la pression p2 se soit établie à nouveau dans le volume de compensation et le volume de référence par l'écoulement sortant de la première quantité de liquide V3.

9.  Procédé selon la revendication 8, dans lequel la première quantité de liquide V3 est définie à partir des pressions p1 et p2 et du contenu de volume Vref du volume de référence, dans lequel s'applique de manière préférée :

$$V3 = Vref * (p1 - p2) / p2.$$

10.  Système médical avec une machine de traitement (10) et un équipement jetable (20) avec des zones de guidage de liquide, lequel peut être accouplé à la machine de traitement, dans lequel l'équipement jetable présente une section de capteur de flux (2), et dans lequel l'équipement jetable présente par ailleurs un volume de compensation (3), lequel est relié et/ou peut être relié de manière fluidique à la section de capteur de flux, dans lequel le volume de compensation peut être accouplé à un volume de référence (4) de la machine de traitement ou dans lequel l'équipement jetable présente un volume de référence, lequel est relié ou peut être relié au volume de compensation par l'intermédiaire d'une liaison sous pression, **caractérisé en ce que** le système médical comprend une pompe, laquelle est présente pour déplacer un liquide médical à l'intérieur du système médical, dans lequel le volume de compensation peut être soumis à l'action d'une pression au moyen de la pompe.

11.  Système médical selon la revendication 10 avec une section de pompe (1), laquelle est reliée ou peut être reliée de manière fluidique au volume de compensation, dans lequel la section de pompe est de manière préférée une section de tuyau flexible pouvant être placé à l'intérieur d'une pompe occlusive de la machine de traitement.

12.  Système médical selon la revendication 10 ou 11, dans lequel la machine de traitement présente au moins un et de manière préférée au moins deux capteurs de pression, par l'intermédiaire duquel ou desquels la pression peut être mesurée dans le volume de compensation et/ou dans le volume de référence, et/ou dans lequel la machine de traitement présente au moins un actionneur de soupape et/ou de pince (C3), par l'intermédiaire duquel une liaison fluidique peut être fermée et ouverte dans l'équipement jetable, par l'intermédiaire de laquelle du liquide peut s'écouler à travers le capteur de flux hors du ou dans le volume de compensation, et/ou dans lequel la machine de traitement présente au moins un actionneur de soupape et/ou de pince, par l'intermédiaire duquel une liaison sous pression entre le volume de compensation et le volume de référence peut être ouverte et fermée, et/ou dans lequel la machine de traitement présente au moins un actionneur de soupape et/ou de pince (C2), par l'intermédiaire duquel une liaison sous pression entre le volume de référence et une zone de pression, en particulier l'atmosphère, peut être ouverte et fermée et/ou dans lequel la machine de traitement présente au moins un capteur de flux, lequel peut être couplé à la section de capteur de flux de l'équipement jetable, dans lequel il s'agit de manière préférée d'un capteur de flux à ultrasons ou d'un capteur de pression différentielle ou d'un capteur électromagnétique.

13.  Système médical, en particulier système médical selon l'une quelconque des revendications 10 à 12, avec une commande servant à évaluer les signaux d'un capteur de flux, dans lequel la commande présente une fonction d'étalonnage et/ou de surveillance pour étalonner et/ou surveiller le capteur de flux selon un procédé selon l'une quelconque des revendications 1 à 9, dans lequel l'étalonnage et/ou la surveillance sont effectués de manière préférée automatiquement par la commande et/ou dans lequel le procédé est mis en oeuvre de manière préférée automatiquement par la commande.

14.  Equipement jetable d'un système médical selon l'une quelconque des revendications 10 à 12.

15.  Machine de traitement d'un système médical selon l'une quelconque des revendications 10 à 12 ou selon la revendication 13 si elle dépend d'une quelconque des revendications 10 - 12.

Fig. 1

Fig. 2a

$C_1$

$$P_o$$
$$V_{ref}$$

~ 4

$C_2$

$$P_1, V_1$$

~ 3

1

$C_3$

offen    zu

Fig. 2b

Fig. 2c

Fig. 2d

Fig. 3a

Fig. 3b

Fig. 3c

Fig. 3d

Fig. 3e

$C_1$

$P_2$
$V_{ref}$  ~ 3

$C_2$

$V_1 +$
$P_2$ $V_2 +$  ~ 4
$V_3$

$C_3$

offen    zu

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2015073604 A1
- EP 2468390 A2

- WO 2015007596 A1